# EUROPEAN PATENT APPLICATION

(11) **EP 4 700 784 A1**
(43) Date of publication of application: **25.02.2026**
(21) Application number: 25196794.9
(22) Date of filing: 19.08.2025
(51) Int. Cl.: G16H 30/40, G16H 40/63, G16H 50/70, A61C 13/00, G06F 3/048

(54) **METHOD AND APPARATUS FOR PROVIDING USER INTERFACE FOR MANUFACTURING DENTAL PROSTHESIS, AND COMPUTER READABLE MEDIUM HAVING PROGRAM FOR PERFORMING THE METHOD**

(30) Priority: 21.08.2024 KR 20240111864; 21.10.2024 WO PCT/KR2024/015963
(71) Applicant: Imagoworks Inc., Seoul 06034 (KR)
(72) Inventor: WANG, Geon, 05675 Seoul (KR); LEE, Junil, 14349 Gwangmyeong-si Gyeonggi-do (KR); KIM, Jiyun, 13522 Seongnam-si Gyeonggi-do (KR); JEONG, Seoyeon, 08366 Seoul (KR); CHOI, Jinhyeok, 06295 Seoul (KR)
(74) Representative: BCKIP Part mbB

(57) **Abstract**

A method for providing a user interface for manufacturing a prosthesis includes identifying teeth from three-dimensional oral data of a patient, displaying a target tooth for a prosthesis manufacturing among the teeth on a screen in a selectable state by a user, displaying a first wheel menu for generation of the prosthesis for the target tooth for dental work or data removal operation, and displaying a first tool menu providing items for the generation of the prosthesis corresponding to the target tooth for dental work on the screen.

## Description

### BACKGROUND

### 1. FIELD

Embodiments relate to method and apparatus for providing a user interface for manufacturing a prosthesis, and a computer readable medium having program for performing the same. More particularly, the method and apparatus for providing the user interface for manufacturing the prosthesis which enable generation and modification of the prosthesis intuitively and conveniently, and the computer readable medium having program for performing the same.

### 2. DESCRIPTION OF THE RELATED ART

A three-dimensional oral data refers to data obtained by scanning teeth and oral cavity using a three-dimensional scanner. A dental treatment such as a prosthetic treatment including in-lay, on-lay, and a crown, an implant and an orthodontic treatment may utilize a patient's oral data for designing prosthesis or implant and manufacturing an orthodontic appliance.

While such dental prosthesis was traditionally manufactured manually, they are now increasingly being designed using three-dimensional CAD software. Each CAD software product requires a user to input different types of data and interact in various ways during a prosthesis design process. To utilize three-dimensional CAD software, the user is required a data for preprocessing of the three-dimensional oral data, a data for the prosthesis design process and finalization tasks after design completion, and a data of an interaction. As an amount of data that the user must input during the prosthesis design process increases, time and resources required for designing the prosthesis have also significantly increased.

### SUMMARY

Embodiments provide a method for providing a user interface for manufacturing a prosthesis with an intuitive and convenient menu.

Embodiments provide an apparatus for providing the user interface for manufacturing the prosthesis.

Embodiments provide computer readable medium having program for performing the same.

A method for providing a user interface for manufacturing a prosthesis includes identifying teeth from a three-dimensional oral data of a patient, displaying a target tooth which requires a prosthesis manufacturing among the teeth on a screen in a selectable state by a user, displaying a first wheel menu for generation of the prosthesis for the target tooth or data removal operation, and displaying a first tool menu providing items for generation of prosthesis corresponding to the target tooth on the screen.

In an embodiment, the displaying the target tooth on the screen in the selectable state by the user may include displaying a tooth identification number of the target tooth in the selectable state in a tooth number component including the tooth identification number of each of the teeth, displaying a three-dimensional image of the three-dimensional oral data on the screen in a three-dimensional rotatable state, and displaying a tooth number label for the tooth identification number of the target tooth included in the three-dimensional image adjacent to the target tooth .

In an embodiment, in the displaying the first tool menu on the screen, when the target tooth is selected by the user, a first item for the tooth identification number of the target tooth and a classification type of the target tooth, a second item for a margin line of the target tooth, a third item for an insertion direction of the prosthesis, and a fourth item for an inner surface of the prosthesis may be displayed in the first tool menu.

In an embodiment, the displaying the first wheel menu on the screen may include displaying a library selection item and a data removal item adjacent to a scan body, generating an artificial abutment model based on values input in the library selection item, and displaying the library selection item, the data removal item, and a prosthesis generation item adjacent to the artificial abutment model.

In an embodiment, in the displaying the first tool menu on the screen, when the scan body is selected by the user, the classification type may be displayed as an implant in the first item, and the first item and the fourth item is active in the selectable state, and the second item and the third item may be inactive in a non-selectable state.

In an embodiment, in the first tool menu on the screen, when the artificial abutment model is selected by the user, the classification type corresponding to the target tooth may be displayed as an implant in the first item, and the first item, the second item, the third item, and the fourth item may be active in the selectable state.

In an embodiment, the method may further include determining a type of the prosthesis corresponding to each of the teeth. And when the prosthesis corresponding to the target tooth is bridge structure, in the determining the type of the prosthesis corresponding to each of the teeth, whether the bridge structure between adjacent teeth among the teeth is connected may be determined, and in the displaying the target tooth on the screen in the selectable state by the user, the target tooth may be displayed in a group selectable state or in an individual selectable state.

In an embodiment, in the displaying the first tool menu on the screen, when a missing tooth combined with the bridge structure is selected by the user, the classification type may be displayed as a pontic in the first item, the first item may be active in the selectable state, and the second item, the third item, and the fourth item may be inactive in a non-selectable state.

In an embodiment, the method may further include generating the prosthesis combined with the target tooth based on a data set in the first wheel menu and the first tool menu, displaying a second wheel menu for data removal operation adjacent to the prosthesis, and displaying a second tool menu providing items for generation and modification of the prosthesis.

In an embodiment, in the displaying the second tool menu, when the prosthesis is selected by the user, a first item for a tooth identification number of the target tooth combined with the prosthesis and a classification type of the prosthesis, a second item for a margin line of the target tooth, a third item for an insertion direction of the prosthesis, a fourth item for an inner surface of the prosthesis, a fifth item for three-dimensional manipulation of the prosthesis, a sixth item for modification of an outer shape of the prosthesis, a seventh item for adjustment between the prosthesis and an adjacent tooth or an opposite tooth of the prosthesis, and an eighth item for a bridge connection of the prosthesis may be displayed in the second tool menu.

An apparatus for providing a user interface for manufacturing a prosthesis includes an input part, a controller, and an output part. The input part may receive a manipulation signal of a user. The controller identifies teeth included in a three-dimensional oral data of a patient, and extracts items for a generation and a modification of the prosthesis corresponding to a target tooth among the teeth, according to the manipulation signal received from the input part. The output part receives an output signal from the controller, and displays a three-dimensional image of the three-dimensional oral data, a wheel menu displayed adjacent to the target tooth, and a tool menu providing the items for the generation or the modification of the prosthesis.

In an embodiment, the wheel menu may include a first wheel menu configured to be displayed adjacent to the target tooth and a second wheel menu configured to be displayed adjacent to the prosthesis combined with the target tooth .

In an embodiment, the tool menu may include a first tool menu configured to provide a first item, a second item, a third item, and a fourth item for the generation of the prosthesis and a second tool menu configured to provide the first item, the second item, the third item, and the fourth item for the generation of the prosthesis, and configured to provide a fifth item, a sixth item, a seventh item, and an eighth item for the modification of the prosthesis.

In an embodiment, the first item provides a modification of a tooth identification number of the target tooth and a classification type of the target tooth or the prosthesis. The second item provides a modification of a margin line of the target tooth . The third item provides a modification of an insertion direction of the prosthesis. the fourth item provides a modification of an inner surface of the prosthesis. The fifth item provides a three-dimensional manipulation of the prosthesis. The sixth item provides a modification of an outer shape of the prosthesis. The seventh item provides a modification for adjustment between the prosthesis and an adjacent tooth of an opposite tooth of the prosthesis. And the eighth item provides a modification for a bridge connection of the prosthesis.

A non-transitory computer-readable storage medium has stored thereon program instructions, the program instructions executable by at least one hardware processor to identify teeth from a three-dimensional oral data of a patient, display a target tooth for a prosthesis manufacturing among the teeth on a screen in a selectable state by a user, display a first wheel menu for generation of the prosthesis for the target tooth or data removal operation, and display a first tool menu providing items for generation of prosthesis corresponding to the target tooth on the screen.

In a method for providing a user interface for manufacturing a prosthesis according to embodiments of the present inventive concept, a first wheel menu for generating the prosthesis or for a data removal operation may be displayed on the screen adjacent to a target tooth for dental work, a first tool menu providing items for generating the prosthesis corresponding to the target tooth for dental work, a second wheel menu for the data removal operation adjacent to the prosthesis, and a second tool menu providing items for generation and modification of the prosthesis may be displayed on the screen. Accordingly, a method process for manufacturing prosthesis may be performed quickly and accurately by using the method for providing the user interface for manufacturing the prosthesis. In addition, a prosthesis manufacturing process may be carried out more efficiently by utilizing the first wheel menu, the second wheel menu, the first tool menu, and the second tool menu. In addition, since the first wheel menu, the second wheel menu, the first tool menu, and the second tool menu provide a more intuitive and consistent user interface for prosthesis manufacturing process, an efficiency of the prosthesis manufacturing process may be improved.

In an apparatus for providing the user interface for manufacturing the prosthesis according to embodiments of the present inventive concept, a controller may identify teeth included in three-dimensional oral data of a patient, automatically determine a type of prosthesis corresponding to each of the teeth, and automatically generate a prosthesis to be combined with the target tooth for dental work based on data set in the first wheel menu and the first tool menu. An output part may receive an output signal from the controller and display the first wheel menu, the second wheel menu, the first tool menu, and the second tool menu on the screen. Accordingly, the user interface and the apparatus for implementing the same may be provided, which enable the prosthesis manufacturing process to be carried out more efficiently.

### BRIEF DESCRIPTION OF THE DRAWINGS

Illustrative, non-limiting embodiments will be more clearly understood from the following detailed description in conjunction with the accompanying drawings.
FIG. 1 is a flowchart illustrating a method for providing a user interface for manufacturing a prosthesis according to an embodiment of the present inventive concept.
FIG. 2 is a block diagram illustrating an apparatus for providing the user interface for manufacturing the prosthesis according to an embodiment of the present inventive concept.
FIGS. 3, 4, 5, and 6 are views for explaining displaying a target tooth for dental work on a screen in a selectable state by a user of FIG. 1.
FIGS. 7 and 8 are views for explaining displaying a first wheel menu adjacent to the target tooth for dental work and displaying a first tool menu providing items for generating the prosthesis of FIG. 1.
FIGS. 9 and 10 are views for explaining displaying the first tool menu providing items for generating the prosthesis of FIG. 1.
FIG. 11 is a view illustrating generating the prosthesis to be combined with the target tooth for dental work, based on data set in the first wheel menu and the first tool menu of FIG. 1.
FIG. 12 is a view explaining for displaying a second wheel menu for data removal operation adjacent to the prosthesis and displaying a second tool menu providing items for generation and modification of the prosthesis of FIG. 1.
FIG. 13 is a view explaining for an example of displaying the second tool menu providing the items for generation and modification of the prosthesis of FIG. 1.
FIG. 14 is a view explaining for another example of displaying the second tool menu providing the items for generation and modification of the prosthesis of FIG. 1.
FIG. 15 is a flowchart illustrating a method for providing a user interface for manufacturing a prosthesis according to another embodiment of the present inventive concept.
FIG. 16 is a flow chart illustrating displaying a first wheel menu adjacent to a scan body or an artificial abutment model of FIG. 15.
FIG. 17 is a flow chart displaying a first tool menu providing items for generating the prosthesis for the scan body or the artificial abutment model of FIG. 15.
FIG. 18 is a view explaining for displaying a library selection item and a data removal item adjacent to the scan body of FIG. 16.
FIG. 19 is a view explaining for displaying each of the library selection item, the data removal item, and a prosthesis generation item, adjacent to the artificial abutment model of FIG. 16.
FIG. 20 is a view explaining for displaying the first tool menu for the scan body of FIG. 17.
FIG. 21 is a view explaining for displaying the first tool meu for the artificial abutment model.
FIG. 22 is a view explaining for displaying the second wheel menu for data removal operation and library selection, adjacent to the prosthesis of FIG. 15.
FIG. 23 is a view explaining for the second tool menu providing items for generation and modification of the prosthesis of FIG. 15.
FIG. 24 is a flowchart illustrating a method for providing a user interface for manufacturing a prosthesis according to still another embodiment of the present inventive concept.
FIG. 25 is a view explaining for displaying a target tooth for dental work on a screen in a group selectable state or in an individual selectable state by a user of FIG. 24.
FIGS. 26 and 27 are views explaining for displaying the first wheel menu adjacent to the target tooth for dental work which is selected either as a group or individually of FIG. 24.
FIG. 28 is a view explaining for displaying the first tool menu providing items for generating the prosthesis of FIG. 24.
FIG. 29 is a view explaining for displaying the second wheel menu for data removal operation adjacent to the prosthesis of FIG. 24.
FIG. 30 is a view explaining for an example of the second tool menu providing items for generation and modification of the prosthesis of FIG. 24.
FIG. 31 is a view explaining for another example of the second tool menu providing items for generation and modification of the prosthesis of FIG. 24.
FIG. 32 is a view explaining for still another example of the second tool menu providing items for generation and modification of the prosthesis of FIG. 24.

### DETAILED DESCRIPTION OF THE EMBODIMENTS

The specific structural or functional descriptions of embodiments of the present inventive concept disclosed herein are merely illustrated for the purpose of explaining the embodiments of the present inventive concept. The embodiments of the present inventive concept may be implemented in various forms and should not be construed as being limited to the embodiments described herein.

The present inventive concept is capable of various modifications and having various forms. Specific embodiments are illustrated in the drawings and described in detail in the text. However, this is not intended to limit the present inventive concept to the specific disclosed forms, and it should be understood to include all modifications, equivalents, and substitutes that fall within the spirit and technical scope of the present inventive concept.

Terms such as first and second may be used to describe various components, but the components should not be limited by these terms. These terms may be used merely to distinguish one component from another component. For example, a first component may be referred to as a second component without departing from the scope of the present inventive concept, and likewise, the second component may also be referred to as the first component.

When a component is referred to as being "connected" or "coupled" to another component, it may be directly connected or coupled to the other component, or there may be another component in between. On the other hand, when a component is referred to as being "directly connected" or "directly coupled" to another component, it should be understood that there is no other component in between. Other expressions used to describe the relationships between components, such as "between" and "immediately between" or "adjacent to" and "directly adjacent to," should be interpreted in the same manner.

The terminology used in this application is merely for the purpose of describing particular embodiments and is not intended to limit the present inventive concept. Unless explicitly stated otherwise, the singular expressions include the plural expressions as well. In this application, the terms such as "include" or "have" are intended to designate that the stated features, numbers, steps, operations, components, parts, or combinations thereof exist, and are not intended to preclude the possibility that one or more other features, numbers, steps, operations, components, parts, or combinations thereof may also exist or be added.

Unless otherwise defined, all terms used herein including technical or scientific terms have the same meaning as commonly understood by one of ordinary skill in the art to which this inventive concept belongs. Terms defined in commonly used dictionaries are to be interpreted as having meanings consistent with their use in the relevant technical field and should not be interpreted in an idealized or overly formal sense unless expressly so defined in this application.

Meanwhile, in cases where an embodiment may be implemented otherwise, functions or operations specified in a specific block may occur in an order different from that specified in the flowchart. For example, two successive blocks may in fact be executed substantially simultaneously, or the blocks may sometimes be executed in reverse order depending on the functions or operations involved.

Hereinafter, preferred embodiments of the present inventive concept will be described in more detail with reference to the accompanying drawings. In the drawings, the same reference numerals denote the same components, and repeated descriptions of the same components will be omitted.

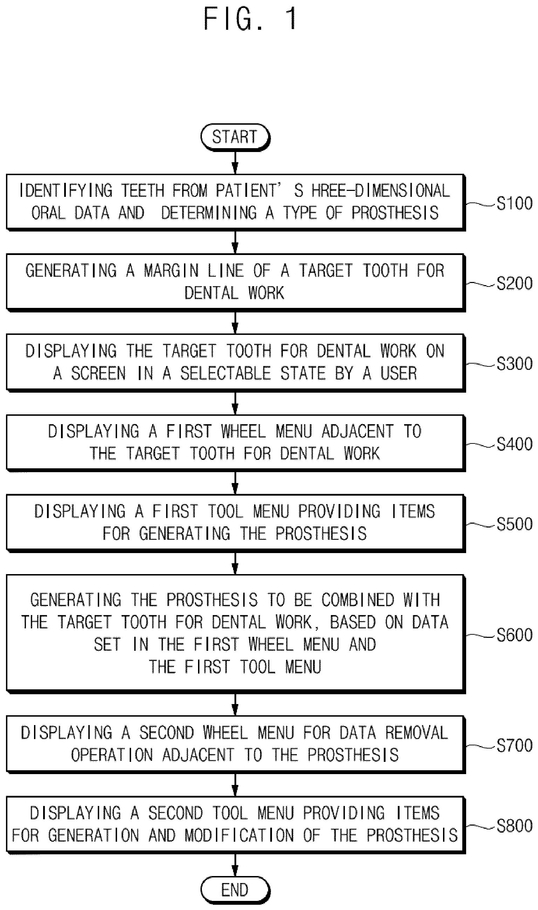

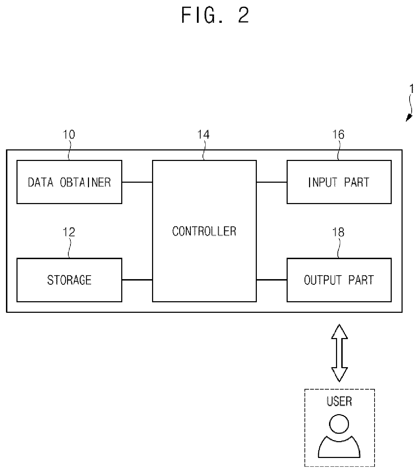

FIG. 1 is a flowchart illustrating a method for providing a user interface for manufacturing a prosthesis according to an embodiment of the present inventive concept. FIG. 2 is a block diagram illustrating an apparatus for providing the user interface for manufacturing the prosthesis according to an embodiment of the present inventive concept.

Referring to FIGS. 1 and 2, a method for providing a user interface for manufacturing a prosthesis according to an embodiment of the present inventive concept may include identifying teeth from a three-dimensional oral data of a patient and determining a type of a prosthesis S100, generating a margin line of a target tooth for dental work S200, displaying the target tooth for dental work on a screen in a selectable state by a user S300, displaying a first wheel menu adjacent to the target tooth for dental work S400, displaying a first tool menu providing items for generating the prosthesis S500, generating the prosthesis to be combined with the target tooth for dental work, based on data set in the first wheel menu and the first tool menu S600, displaying a second wheel menu for data removal operation adjacent to the prosthesis S700, and displaying a second tool menu providing items for generation and modification of the prosthesis S800.

In an embodiment, the identifying teeth from the three-dimensional oral data of the patient and the determining the type of the prosthesis S100, the generating the margin line of the target tooth for dental work S200, the displaying the target tooth for dental work on the screen in the selectable state by a user S300, the displaying the first wheel menu adjacent to the target tooth for dental work S400, and displaying the first tool menu providing items for generating the prosthesis S500 may be performed after the generating the prosthesis to be combined with the target tooth for dental work, based on the data set in the first wheel menu and the first tool menu S600 is performed.

In an embodiment, the displaying the second wheel menu for the data removal operation adjacent to the prosthesis S700, and the displaying the second tool menu providing the items for generation and modification of the prosthesis S800 may be performed after the generating the prosthesis to be combined with the target tooth for dental work, based on the data set in the first wheel menu and the first tool menu S600.

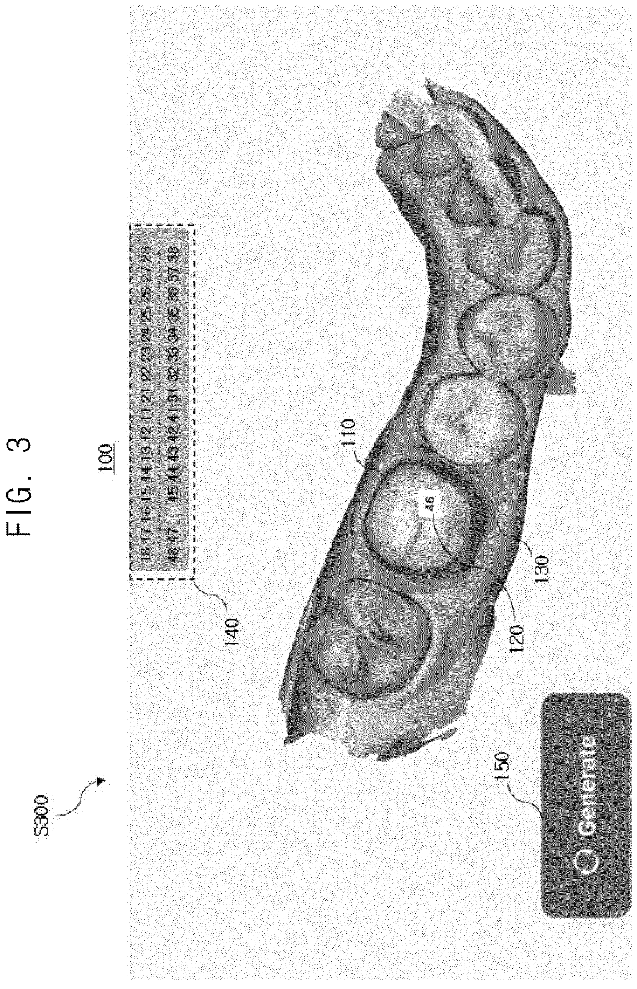

The method for providing the user interface for manufacturing the prosthesis (e.g., the method for providing the user interface for manufacturing the prosthesis according to FIGS. 1, 15, and 24) according to an embodiment of the present inventive concept may be performed by the apparatus for providing the user interface for manufacturing the prosthesis 1. For example, the method for providing the user interface for manufacturing the prosthesis may be performed by a computing apparatus. In an embodiment, the computing apparatus may be a mobile phone, video phone, smart pad, tablet PC, desktop computer, or laptop for providing the user interface (e.g., the user interface 100 in FIG. 3) to the user. However, a type of the apparatus for providing the user interface for manufacturing the prosthesis 1 according to embodiments of the inventive concept may not be limited thereto.

The apparatus for providing the user interface for manufacturing the prosthesis 1 according to an embodiment of the inventive concept may include a data obtainer 10, a storage 12, a controller 14, an input part 16, and an output part 18.

In an embodiment, the apparatus for providing the user interface for manufacturing the prosthesis 1 may be an electronic apparatus that implements the user interface via a computer-aided design (CAD) program for dental use. The apparatus for providing the user interface for manufacturing the prosthesis 1 according to an embodiment may perform a CAD process to assist actual dental treatment, such as prosthodontic treatment or implantation. The CAD process may be a series of processes in which the three-dimensional oral data of a patient is obtained, a virtual tooth model is retrieved from a library under control of a computer program, and the virtual model is arranged in a target location of the three-dimensional oral data. The three-dimensional oral data may be data including a three-dimensional data of teeth including a damaged target tooth for dental work, and the target location may be a location of a tooth arranged in the dental arch included in the three-dimensional oral data.

The data obtainer 10 may obtain the three-dimensional oral data including the damaged target tooth for dental work of the patient. For example, the three-dimensional oral data may be obtained by scanning a plaster model formed by taking an impression of the patient's oral cavity using a 3D scanner. In another example, the three-dimensional oral data may be obtained by scanning the inside of the patient's oral cavity using a 3D intra-oral scanner. In still another example, the three-dimensional oral data may be obtained by generating head cross-sectional images of the patient using computed tomography (CT), segmenting tooth boundaries from the respective images, and integrating the segmented data. The three-dimensional oral data may include images obtained by capturing the maxillary teeth from below with the mouth open, images of the mandibular teeth from above with the mouth open, localized images with the mouth closed, oral radiographs, and the like. The obtained three-dimensional oral data may be stored in the storage 12.

In an embodiment, the three-dimensional oral scan data may be a mesh data including three-dimensional vertices and triangle or rectangle surfaces generated by connecting the vertices. In addition, the three-dimensional oral scan data may be video data captured through a three-dimensional scanner. A file format of the three-dimensional oral scan data may not be limited, for example, the file format may be one among ply, obj, or stl. Each of the CT data and the MRI data may include a two-dimensional image data and three-dimensional volume data.

The storage 12 may store various data including a data necessary for the operation of the apparatus for providing the user interface 1 and a data generated during the operation. In an embodiment, the storage 12 may store the three-dimensional oral data of individual patients, and in a dental treatment simulation process, the three-dimensional oral data of a specific patient may be provided to the controller 14 in response to a user request. For example, the image of the upper dental arch and the image of the lower dental arch stored in the storage 12 may be provided to the controller 14 according to a user request.

The controller 14 controls functional parts (e.g., the data obtainer 10, storage 12, input part 16, and output part 18) while performing the CAD process under control of a computer program. The controller 14 displays the three-dimensional oral data of the patient on a screen through the output part 18. For example, the controller 14 may display, through the output part 18, the normal teeth, the target tooth for dental work to be treated, and the contralateral tooth corresponding to the location of the target tooth for dental work.

In an embodiment, the controller 14 may include an artificial neural network that performs the identifying teeth from the three-dimensional oral data of the patient and determining the type of the prosthesis S100, and the generating the margin line of the target tooth for dental work S200. Specifically, the controller 14 may extract the teeth from the three-dimensional oral data of the patient, determine an identification number corresponding to each of the teeth, and distinguish the target tooth for dental work among the teeth, thereby automatically determining a type of prosthesis to be used for the target tooth for dental work. In addition, the controller 14 may automatically generate the margin line of the target tooth for dental work.

The input part 16 may receive a manipulation signal from the user. For example, the input part 16 may receive a command signal based on user actions such as selecting a specific area in the three-dimensional oral data or selecting menus displayed on the output part 18. In an embodiment, the input part 16 may be an input device such as a mouse or touch panel for transmitting the command signals to the controller 14. However, a type of the input part 16 according to embodiments of the inventive concept may not be limited thereto.

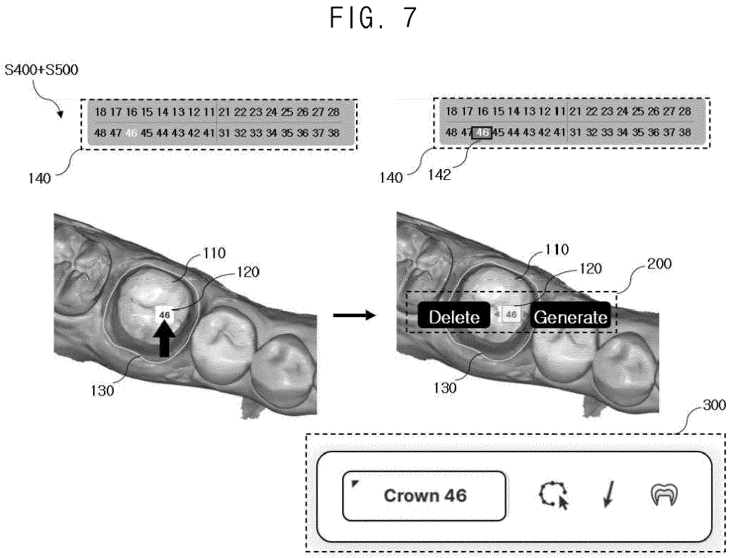

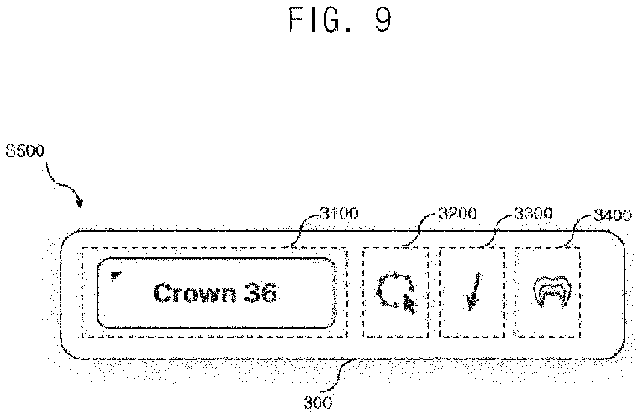

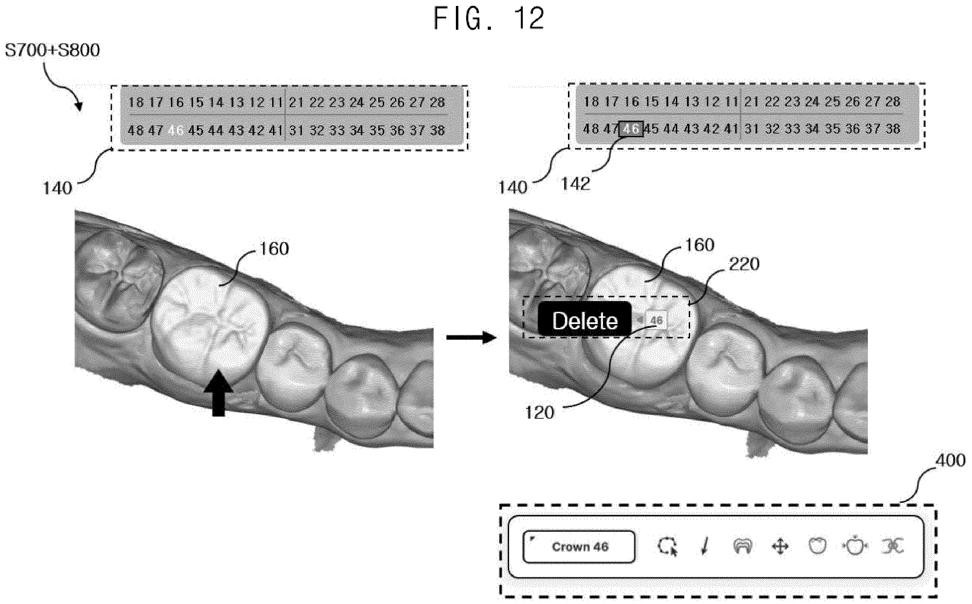

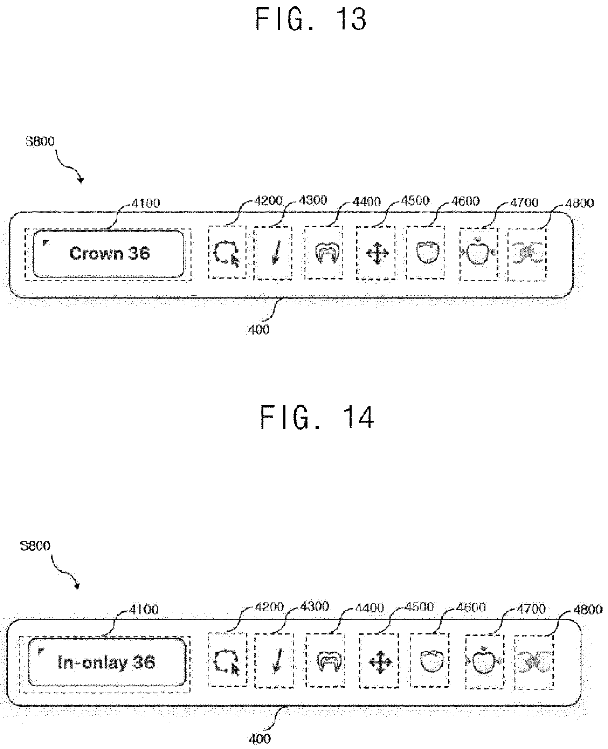

The controller 14 may extract items for generating and modifying the prosthesis corresponding to the target tooth for dental work among the teeth, according to the manipulation signal received from the input part 16. The output part 18 may receive an output signal from the controller 14 and display a tool menu using the extracted items. Specifically, the output part 18 may receive the output signal from the controller 14 and display a three-dimensional image of the three-dimensional oral data, wheel menus (e.g., the first wheel menu 200 of FIG. 7 and the second wheel menu 220 of FIG. 12) displayed adjacent to the target tooth for dental work or the prosthesis, and tool menus (e.g., the first tool menu 300 of FIG. 9 and the second tool menu 400 of FIG. 13) that provide items for generation or modification of the prosthesis.

The output part 18 may include the screen for displaying the user interface. In an embodiment, the output part 18 may be a television for displaying the user interface, or may include a computer monitor, laptop screen, tablet screen, and the like. However, a type of the output part 18 according to embodiments of the inventive concept may not be limited thereto.

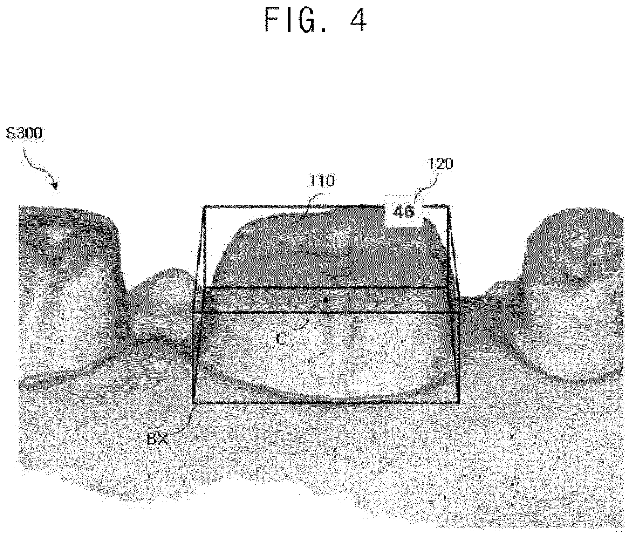

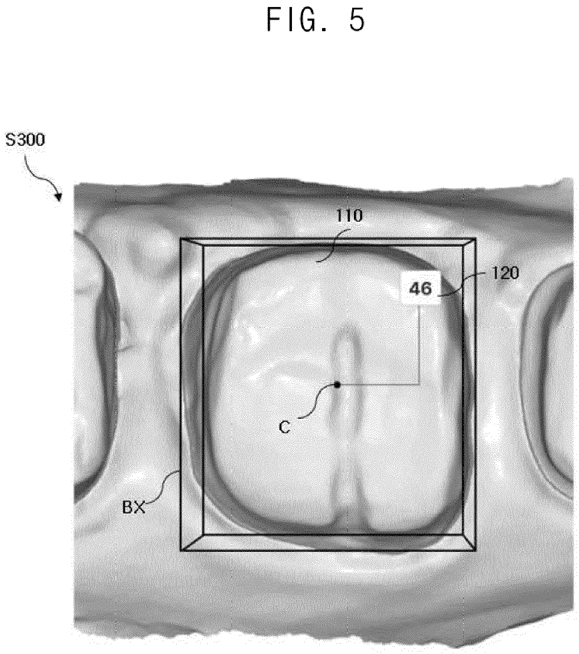

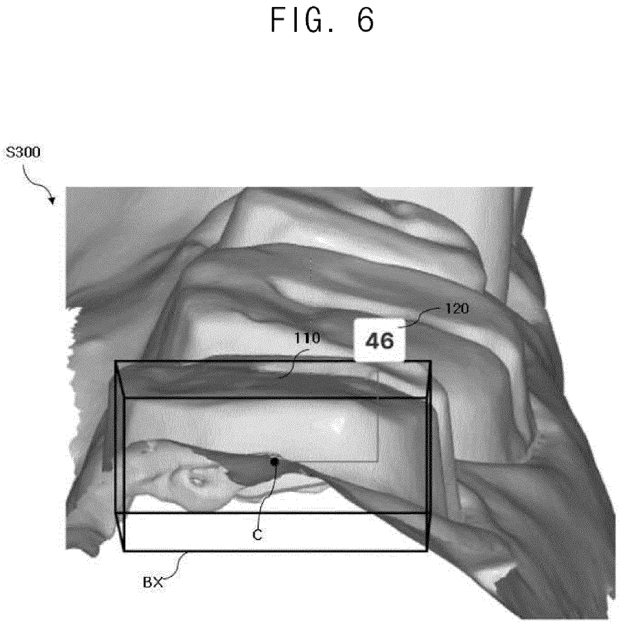

FIGS. 3, 4, 5, and 6 are views for explaining displaying a target tooth for dental work on a screen in the selectable state by a user of FIG. 1. For example, FIGS. 4, 5, and 6 are views illustrating the target tooth for dental work in the user interface 100 of FIG. 3, rotated in three dimensions in order to observe the target tooth for dental work from a specific angle.

Referring to FIGS. 1, 2, 3, 4, 5, and 6, in the displaying the target tooth for dental work 110 on a screen in the selectable state by the user, the user interface 100 may include the target tooth for dental work 110, a tooth number label 120, a margin line 130 of the target tooth for dental work 110, a tooth number component 140, and a batch generation button 150.

A three-dimensional image of three-dimensional oral data of the patient may be displayed on a screen where the user interface 100 is shown. For example, the three-dimensional image may be rotatable in three dimensions according to a user operation. The target tooth for dental work 110 may be a tooth among the teeth included in three-dimensional oral data of the patient for which the prosthesis is to be manufactured. For example, the target tooth for dental work 110 may correspond to a prepared tooth, a scan body for an implant, or a missing tooth. The target tooth for dental work 110 may be included in the three-dimensional image.

Here, the prepared tooth may refer to a tooth prepared for a prosthesis (e.g., a crown), and the prepared tooth may refer to a tooth partially ground. Specifically, in order to generate a single crown, a process of removing an entire natural tooth to create a single crown, making placing the prosthesis easier is required, and the natural tooth that undergo the process may be referred to as the prepared tooth. Here, the scan body for the implant may refer to a provisional abutment placed in a gum for implantation, in which type and material of the abutment model are not yet determined. Specifically, after determining the type and material of an abutment model, the scan body may be replaced with the abutment model, and the abutment and the crown may be combined to perform the implant treatment. Here, the missing tooth may refer to a portion of the dental arch where a tooth is lost.

The target tooth for dental work 110 may be displayed in the selectable state by the user on the screen where the user interface 100 is shown. For example, a tooth identification number of the target tooth for dental work 110 may be displayed in the selectable state on the screen in the tooth number component 140. In addition, the target tooth for dental work 110 may be displayed in the selectable state by the user on the screen in the three-dimensional image. Accordingly, the user may select the image of the target tooth for dental work 110 in the three-dimensional image or select a working area 142 including the tooth identification number of the target tooth for dental work 110 in the tooth number component 140.

The tooth number label 120 may be displayed adjacent to the target tooth for dental work 110. The tooth number label 120 may display the tooth identification number of the target tooth for dental work 110 as a numeral. In an embodiment, the tooth number label 120 may be displayed at the upper right of a center point C of a three-dimensional box BX surrounding the boundary of the target tooth for dental work 110. In addition, the tooth number label 120 may be displayed in a fixed location at an upper right of the center point C of the three-dimensional box BX regardless of rotation angle and rotation direction of the three-dimensional image. However, a location of the tooth number label 120 according to embodiments of the inventive concept may not be limited thereto, and the tooth number label 120 may have various locational relationships with the center point C of the three-dimensional box BX.

In an embodiment, the three-dimensional box BX may be a virtual box that is not displayed on the screen. For example, the three-dimensional box BX may be a virtual box generated by the controller 14 to define the area including the target tooth for dental work 110. However, the three-dimensional box BX displayed in the user interface 100 according to embodiments of the inventive concept may not be limited thereto, and the three-dimensional box BX may be displayed on the screen so that the user may visually recognize the three-dimensional box BX.

The margin line 130 may be extracted from three-dimensional oral data of the patient by the controller 14 and may be displayed on the screen through the output part 18. The margin line 130 may be a boundary line indicating an edge portion of the target tooth for dental work 110. For example, the margin line 130 may be a boundary between the prepared tooth and the gum. In order to distinguish the margin line 130 from the dental arch in the three-dimensional image of the three-dimensional oral data, the margin line 130 may be displayed on the screen in a color different from a color of the dental arch.

The tooth number component 140 may display the tooth identification numbers of each of the teeth included in three-dimensional oral data of the patient. For example, the controller 14 may determine the tooth identification number of each of the teeth from the three-dimensional oral data and display the tooth identification number on the screen through the tooth number component 140. In other words, the tooth number component 140 may be an interface menu for conveniently providing the tooth identification numbers of the teeth included in the three-dimensional oral data.

The tooth number component 140 may display the tooth identification number of the target tooth for dental work 110, which requires dental treatment, in the selectable state by the user. For example, the tooth number component 140 may display the target tooth for dental work 110 in an activated state that is selectable on the screen. Specifically, the tooth identification number of the target tooth for dental work 110 may be displayed in a color different from a color of other teeth that do not require treatment in the tooth number component 140. Accordingly, the user may identify the identification number of the target tooth for dental work 110 requiring dental treatment or select the working area 142 through the tooth number component 140.

The batch generation button 150 may be a menu for generating the prosthesis corresponding to the target tooth for dental work 110 at once. For example, when a number of a target teeth for dental work 110 is plurality, the user may press the batch generation button 150 so that prostheses corresponding to the target teeth for dental work 110 are generated and displayed together on the screen. In another example, when a number of target tooth for dental work 110 is only one, a prosthesis corresponding to the single target tooth for dental work 110 may be generated and displayed on the screen through the batch generation button 150. Specifically, the controller 14 may automatically pre-generate data regarding a type of prosthesis corresponding to the target tooth for dental work 110, and when the user selects the batch generation button 150, the controller 14 may transmit an output signal to the output part 18 so that the prosthesis corresponding to the target tooth for dental work 110 is displayed on the screen in a state coupled to the target tooth for dental work 110.

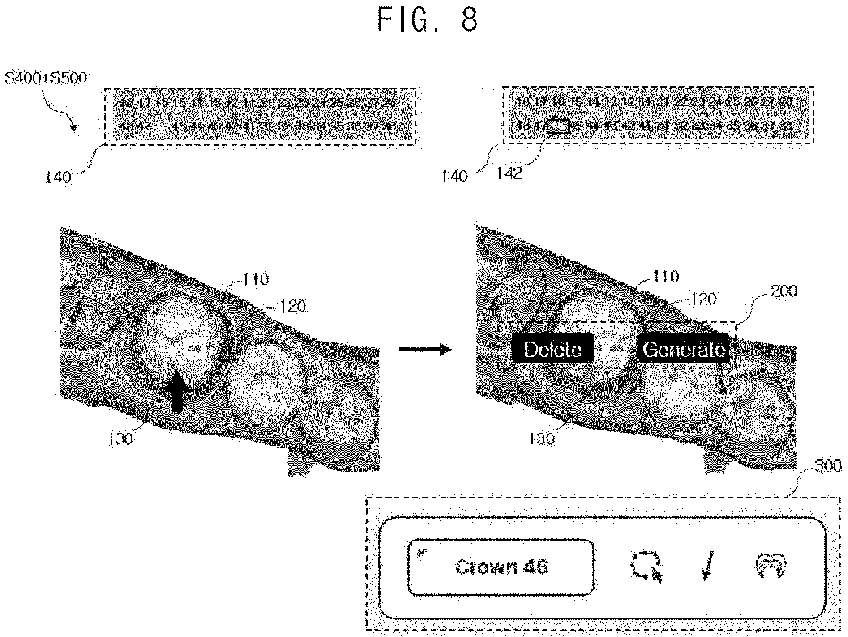

FIGS. 7 and 8 are views for explaining displaying a first wheel menu adjacent to the target tooth for dental work and displaying a first tool menu providing items for generating the prosthesis of FIG. 1.

Referring to FIGS. 7 and 8, in the displaying the first wheel menu 200 adjacent to the target tooth for dental work 110 S400, when the user selects the target tooth for dental work 110, the first wheel menu 200 may be displayed adjacent to the target tooth for dental work 110. In an embodiment, the user may directly select the image of the target tooth for dental work 110 displayed in the three-dimensional image so that the first wheel menu 200 is displayed on the screen. In another embodiment, the user may select the working area 142 including the tooth identification number of the activated target tooth for dental work 110 in the tooth component 140 so that the first wheel menu 200 is displayed on the screen.

The first wheel menu 200 may include items for performing an operation of generating the prosthesis corresponding to the target tooth for dental work 110 or removing data regarding the target tooth for dental work 110. For example, when the target tooth for dental work 110 selected by the user is a prepared tooth, the first wheel menu 200 may provide a prosthesis generation item and a data removal item.

The prosthesis generation item may be an item for generating the prosthesis corresponding to the selected target tooth for dental work 110. While the batch generation button (e.g., the batch generation button 150 in FIG. 3) performs a task of generating prostheses corresponding to all of target teeth for dental work 110requiring work, the prosthesis generation item of the first wheel menu 200 may perform a task of generating a prosthesis corresponding to only one selected target tooth for dental work 110. Although the first wheel menu 200 is described as displaying two items, a number of items displayed in the first wheel menu 200 according to the embodiments of the present inventive concept may not be limited thereto, and various numbers of items or one item may be displayed depending on the type of the selected target tooth for dental work 110.

The data removal item may be an item for removing data set for the tooth classified as the target tooth for dental work 110 among the teeth included in the three-dimensional oral data by the controller 14. For example, when the user selects the data removal item, data on the type of prosthesis corresponding to the target tooth for dental work 110, data on parameters for generating the prosthesis, and the like may be removed, and the target tooth for dental work 110 on which the data removal operation is performed may be deactivated in a state in which work is impossible.

As described above, the first wheel menu 200 may be displayed adjacent to the target tooth for dental work 110. For example, the first wheel menu 200 may be displayed around the tooth number label 120. Specifically, when the selected target tooth for dental work 110 is a prepared tooth, the prosthesis generation item and the data removal item may be displayed on either side of the tooth number label 120.

In the displaying the first tool menu 300 providing items for generating the prosthesis S500, when the user selects the target tooth for dental work 110, the first tool menu 300 may be displayed on the screen. In an embodiment, the user may directly select the image of the target tooth for dental work 110 displayed in the three-dimensional image so that the first tool menu 300 is displayed on the screen. In another embodiment, the user may select the working area 142 including the tooth identification number of the activated target tooth for dental work 110 in the tooth component 140 so that the first tool menu 300 is displayed on the screen.

In an embodiment, when the user selects the target tooth for dental work 110, both of the first wheel menu 200 and the first tool menu 300 may be displayed on the screen. In other words, the apparatus for providing the user interface (e.g., the apparatus for providing the user interface 1 in FIG. 2) may receive the user's operation signal selecting the target tooth for dental work 110, and may simultaneously perform the displaying the first wheel menu 200 adjacent to the target tooth for dental work 110 S400 and the displaying the first tool menu 300 providing items for generating the prosthesis S500.

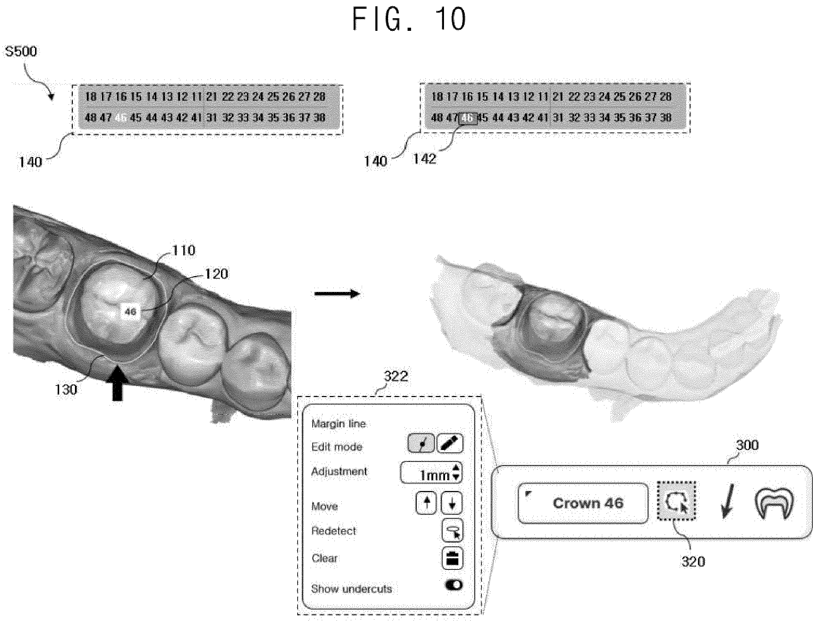

FIGS. 9 and 10 are views for explaining displaying the first tool menu providing items for generating the prosthesis of FIG. 1.

Referring to FIGS. 9 and 10, in the displaying the first tool menu 300 S500, the first tool menu 300 may include a first item 3100, a second item 3200, a third item 3300, and a fourth item 3400. The first item 3100 may be an item for the tooth identification number and classification type of the target tooth for dental work 110. For example, when the user selects the first item 3100, the user may modify each of the selected tooth identification number and the classification type of the target tooth for dental work 110. The classification type displayed in the first item 3100 may provide the user with classification data about the target tooth for dental work 110 before the prosthesis is generated.

The classification type of the target tooth for dental work 110 displayed in the first item 3100 may vary depending on the type of the target tooth for dental work 110. In an embodiment, the classification type displayed in the first item 3100 may include a crown, an implant, and a pontic. In an embodiment, when the target tooth for dental work 110 selected by the user is a prepared tooth, the classification type corresponding to the target tooth for dental work 110 in the first item 3100 may be displayed as a crown. However, classification types displayed in the first item 3100 according to the embodiments of the present inventive concept may not be limited thereto.

The second item 3200 may be an item for the margin line 130 of the target tooth for dental work 110. For example, the second item 3200 may be an item for modifying location, height, shape, and the like of the margin line 130. Specifically, when the user directly selects the second item 3200, a margin line editing menu 322 for displaying items such as moving the margin line 130, re-detecting the margin line 130, removing the margin line 130, and marking an undercut may be displayed on the screen. In addition, when the user selects the image of the margin line 130 in the three-dimensional image, the margin line editing menu 322 displayed on the screen may be displayed. Accordingly, the user may modify the data for the margin line 130 of the selected target tooth for dental work 110 by using the margin line editing menu 322.

The third item 3300 may be an item for an insertion direction of the prosthesis corresponding to the target tooth for dental work 110. When the user selects the third item 3300, the user may modify data for the insertion path of the prosthesis to be combined with the selected target tooth for dental work 110.

The fourth item 3400 may be an item for an inner surface of the prosthesis. For example, the fourth item 3400 may be an item for modifying the processing method of the inner surface of the prosthesis, a space for accommodating an adhesive between the tooth and the prosthesis, a minimum thickness of the prosthesis to prevent fracture, a width of the margin line, an angle inclined toward an occlusal surface from the margin line, and the like. When the user selects the fourth item 3400, the user may modify data for the inner surface of the prosthesis to be combined with the selected target tooth for dental work 110.

In an embodiment, when the target tooth for dental work 110 selected by the user is a prepared tooth, each of the first item 3100, the second item 3200, the third item 3300, and the fourth item 3400 may be activated to be in the selectable state.

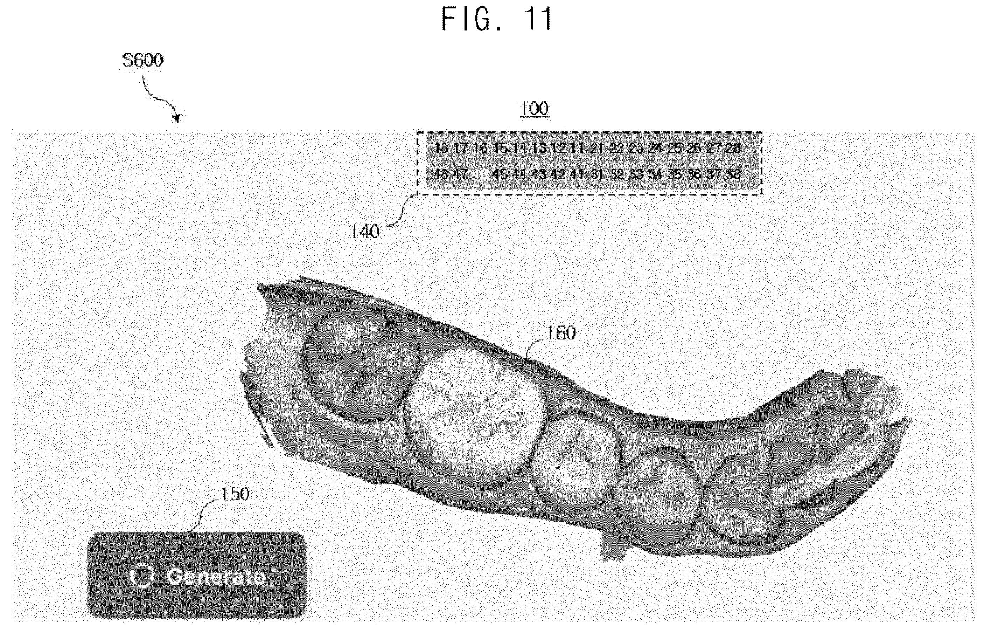

FIG. 11 is a view illustrating generating the prosthesis to be combined with the target tooth for dental work, based on data set in the first wheel menu and the first tool menu of FIG. 1.

Referring to FIGS. 7, 8, and 11, when the user selects the prosthesis generation item of the first wheel menu 200 or selects the batch generation button 150, the generating the prosthesis 160 to be combined with the target tooth for dental work 110 based on the data set in the first wheel menu 200 and the first tool menu 300 may be performed. Accordingly, the target tooth for dental work 110 may be displayed in a state combined with the prosthesis 160 in the user interface 100. The prosthesis 160 may include a crown, an in-lay, an on-lay, a bridge, and the like. The prosthesis 160 may be displayed on the screen in a state selectable by the user.

FIG. 12 is a view explaining for displaying a second wheel menu for data removal operation adjacent to the prosthesis and displaying a second tool menu providing items for generation and modification of the prosthesis of FIG. 1.

Referring to FIG. 12, in the displaying the second wheel menu 220 for the data removal operation adjacent to the prosthesis 160 S700, the second wheel menu 220 may be displayed adjacent to the prosthesis 160 when the user selects the prosthesis 160. In an embodiment, the user may directly select the image of the prosthesis 160 displayed in the three-dimensional image so that the second wheel menu 220 is displayed on the screen. In another embodiment, the user may select the working area 142 including the tooth identification number of the target tooth for dental work (e.g., the target tooth for dental work 110 of FIG. 3) combined with the activated prosthesis 160 in the tooth identification component 140 so that the second wheel menu 220 is displayed on the screen.

The second wheel menu 220 may include items for performing an operation of removing data for the prosthesis 160 and the target tooth for dental work combined with the prosthesis 160. For example, when the prosthesis 160 selected by the user is a single crown combined with a prepped tooth, the second wheel menu 220 may provide a data removal item. Specifically, the data removal item may be an item for removing the data set by the controller (e.g., the controller 14 of FIG. 2) for the tooth classified as the target tooth for dental work among the teeth included in the three-dimensional oral data and the data set for the prosthesis 160 combined with the target tooth for dental work. When the user selects the data removal item, the data for the type of the prosthesis 160 combined with the target tooth for dental work, the parameters for generating the prosthesis 160, the shape of the prosthesis 160, and the like may be removed, and the prosthesis 160 subjected to the data removal operation may be deactivated in a state in which work is not possible.

As described above, the second wheel menu 220 may be displayed on the screen adjacent to the prosthesis 160. For example, the second wheel menu 220 may be displayed around the tooth number label 120 of the target tooth for dental work combined with the prosthesis 160. Specifically, when the selected prosthesis 160 is a single crown, the data removal item may be displayed on a left side of the tooth number label 120.

In the displaying the second tool menu 400 providing items for generation and modification of the prosthesis 160 S800, the second tool menu 400 may be displayed on the screen when the user selects the prosthesis 160. In an embodiment, the user may directly select the image of the prosthesis 160 displayed in the three-dimensional image so that the second tool menu 400 is displayed on the screen. In another embodiment, the user may select the working area 142 including the tooth identification number of the target tooth for dental work 110 combined with the activated prosthesis 160 in the tooth identification component 140 so that the second tool menu 400 is displayed on the screen.

In an embodiment, when the user selects the prosthesis 160, both of the second wheel menu 220 and the second tool menu 400 may be displayed on the screen. In other words, the apparatus for providing the user interface (e.g., the apparatus for providing the user interface 1 of FIG. 2) may receive an operation signal of the user for selecting the prosthesis 160 and simultaneously perform the displaying the second wheel menu 220 for the data removal operation adjacent to the prosthesis 160 S700 and the displaying the second tool menu 400 providing the items for generation and modification of the prosthesis 160 S800.

FIG. 13 is a view explaining for an example of displaying the second tool menu providing the items for generation and modification of the prosthesis of FIG. 1. FIG. 14 is a view explaining for another example of displaying the second tool menu providing the items for generation and modification of the prosthesis of FIG. 1.

Referring to FIGS. 12, 13, and 14, in the displaying the second tool menu 400 providing items for generation and modification of the prosthesis 160 S800, the second tool menu 400 may include a first item 4100, a second item 4200, a third item 4300, a fourth item 4400, a fifth item 4500, a sixth item 4600, a seventh item 4700, and an eighth item 4800. The first item 4100 may be an item for the tooth identification number of the target tooth for dental work combined with the prosthesis 160 and the classification type of the prosthesis 160. For example, when the user selects the first item 4100, the user may modify each of the tooth identification number and the classification type of the prosthesis 160. A classification type data for the prosthesis 160 after generation of the prosthesis may be provided to the user through the classification type in the first item 4100.

Depending on the type of the prosthesis 160, the classification type displayed in the first item 4100 may differ. In an embodiment, the classification type displayed in the first item 4100 may include a crown, in-onlay, implant, pontic, bridge, and the like. In an embodiment, when the prosthesis 160 selected by the user is a single crown, the classification type displayed in the first item 4100 may be a crown. Also, when the prosthesis 160 selected by the user is an in-lay or on-lay, the classification type displayed in the first item 4100 may be an in-onlay. However, the classification type displayed in the first item 4100 according to the embodiments of the present inventive concept may not be limited thereto.

The second, third, and fourth items 4200, 4300, and 4400, which are described with reference to FIGS. 13 and 14, may be substantially a same or similar to the second, third, and fourth items 3200, 3300, and 3400 described with reference to FIGS. 9 and 10. For example, the second item 4200 may be an item for the margin line 130 of the target tooth for dental work combined with the prosthesis 160. The third item 4300 may be an item for the insertion direction of the prosthesis 160 combined with the target tooth for dental work. The fourth item 4400 may be an item for the inner surface of the prosthesis 160. The user may select each of the second, third, and fourth items 4200, 4300, and 4400 to modify the data set for each of the second, third, and fourth items 4200, 4300, and 4400. As the data are modified, the bonding state, shape, and the like of the prosthesis 160 and the target tooth for dental work displayed on the screen may be changed.

The fifth item 4500 may be an item for three-dimensional manipulation of the prosthesis 160. For example, when the user selects the fifth item 4500, the user may perform operations such as moving the prosthesis 160 in a specific direction, rotating the prosthesis 160 at a specific angle, or adjusting the scale of the prosthesis 160. However, operations performed via the fifth item 4500 according to the embodiments of the present inventive concept may not be limited thereto.

The sixth item 4600 may be an item for modifying the outer shape of the prosthesis 160. For example, when the user selects the sixth item 4600, the user may perform operations such as modifying a shape of a specific portion of the prosthesis 160 to be flat, convex, or concave, or adjusting a texture to make the prosthesis 160 smooth. However, operations performed via the sixth item 4600 according to the embodiments of the present inventive concept may not be limited thereto.

The seventh item 4700 may be an item for adjusting between the prosthesis 160 and the adjacent tooth or the opposing tooth. For example, when the user selects the seventh item 4700, the user may perform operations such as setting a distance between the prosthesis 160 and the adjacent tooth or the opposing tooth, copying the shape of the adjacent tooth or the opposing tooth, or removing an overlapping mesh on the occlusal surface of the prosthesis 160. However, operations performed via the seventh item 4700 according to the embodiments of the present inventive concept may not be limited thereto.

The eighth item 4800 may be an item for bridging the prosthesis 160. For example, when the user selects the eighth item 4800, the user may perform operations such as modifying the shape or size of the connecting portion of a bridge structure or selecting a cross-sectional view of the connecting portion. In an embodiment, the eighth item 4800 may be activated in the selectable state only when the selected prosthesis 160 is a bridge structure.

In an embodiment, when the prosthesis 160 selected by the user is a single crown or in-onlay, each of the first, second, third, fourth, fifth, sixth, and seventh items 4100, 4200, 4300, 4400, 4500, 4600, and 4700 may be activated in the selectable state. In an embodiment, when the prosthesis 160 selected by the user is a single crown or in-onlay, the eighth item 4800 may be deactivated in a non-selectable state.

As described above, in the method providing the user interface for manufacturing the prosthesis according to embodiments of the present inventive concept, the first wheel menu 200 for generating or removing data of the prosthesis 160 adjacent to the target tooth for dental work 110, the first tool menu 300 that provides items for generating the prosthesis 160 corresponding to the target tooth for dental work 110, the second wheel menu 220 for removing data adjacent to the prosthesis 160, and the second tool menu 400 that provides items for generating and modifying the prosthesis 160 may be displayed on the screen. Accordingly, the prosthesis fabrication process may be carried out quickly and accurately using the user interface providing method for prosthesis fabrication. Also, the prosthesis fabrication process may be carried out more quickly by using the first wheel menu 200, the second wheel menu 220, the first tool menu 300, and the second tool menu 400. In addition, since the first wheel menu 200, the second wheel menu 220, the first tool menu 300, and the second tool menu 400 provide a more intuitive and consistent user interface 100 for prosthesis fabrication, an efficiency of the prosthesis fabrication process may be improved.

In the apparatus for providing the user interface for manufacturing the prosthesis 1 according to embodiments of the present inventive concept, the controller 14 may identify the teeth included in three-dimensional oral data of the patient, automatically determine the types of prostheses corresponding to each of the teeth, automatically generate the prosthesis 160 combined with the target tooth for dental work 110 based on the data set in the first wheel menu 200 and the first tool menu 300, and the output unit 18 may receive an output signal from the controller 14 and display the first wheel menu 200, the second wheel menu 220, the first tool menu 300, and the second tool menu 400 on the screen. Accordingly, a user interface 100 and apparatus capable of more efficiently performing the prosthesis fabrication process may be provided.

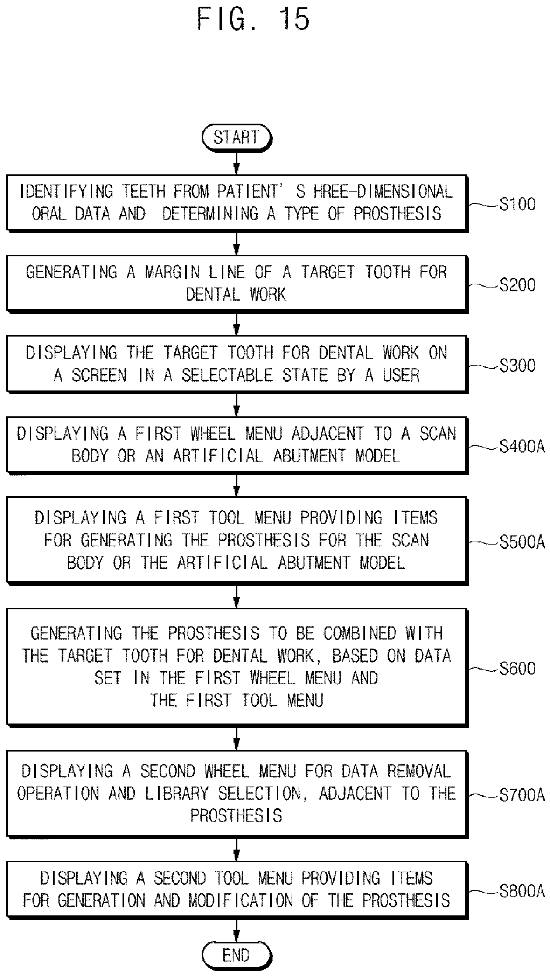

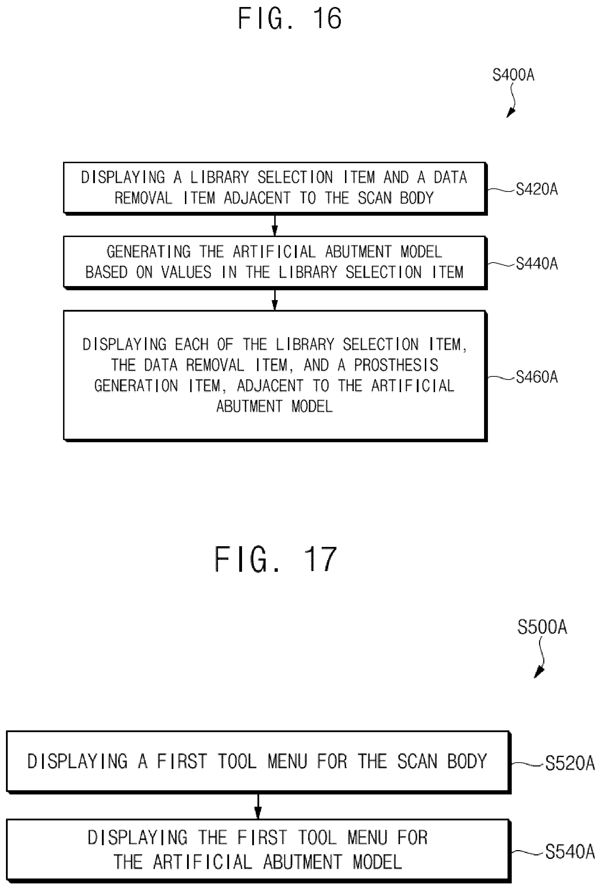

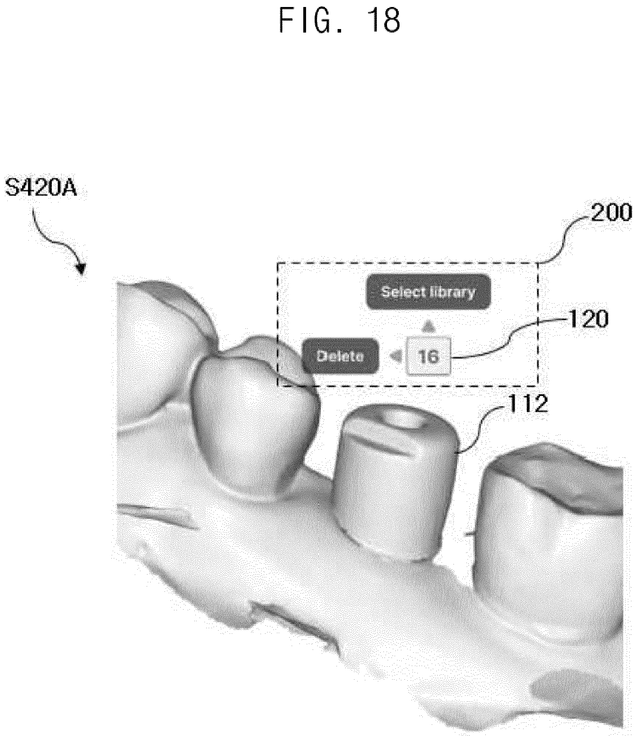

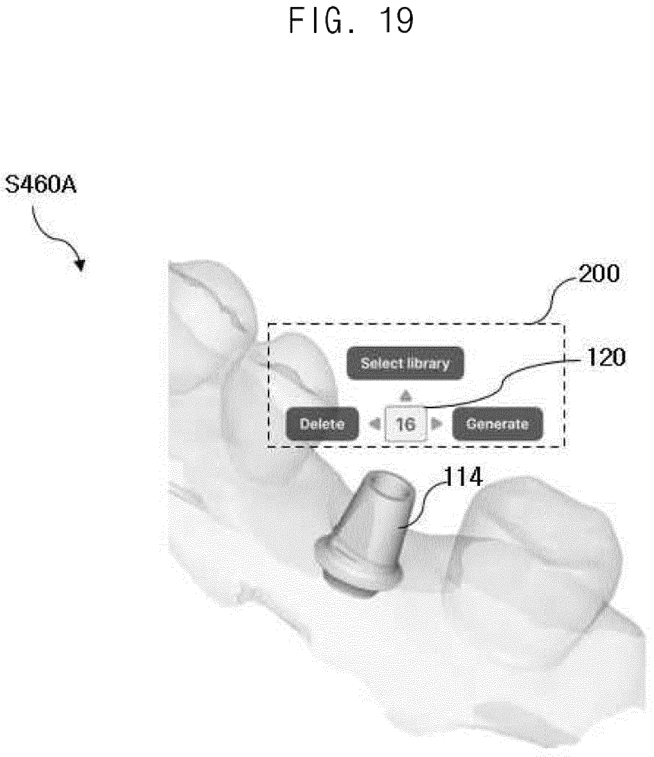

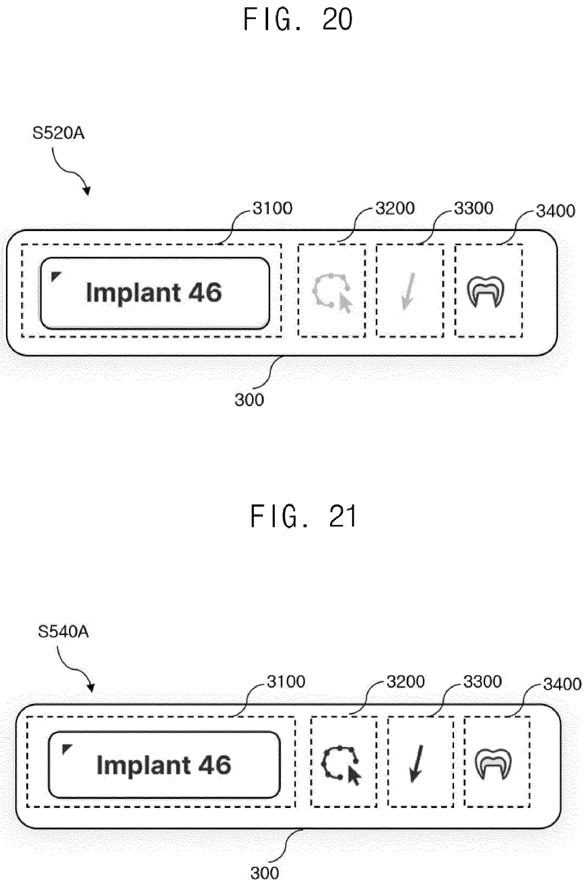

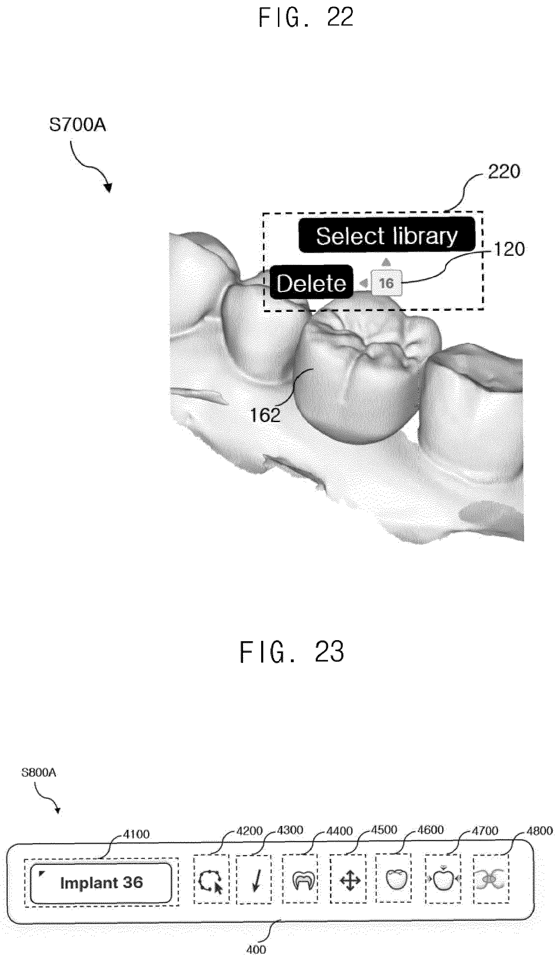

FIG. 15 is a flowchart illustrating a method for providing a user interface for manufacturing a prosthesis according to another embodiment of the present inventive concept. FIG. 16 is a flow chart illustrating displaying a first wheel menu adjacent to a scan body or an artificial abutment model of FIG. 15. FIG. 17 is a flow chart displaying a first tool menu providing items for generating the prosthesis for the scan body or the artificial abutment model of FIG. 15. FIG. 18 is a view explaining for displaying a library selection item and a data removal item adjacent to the scan body of FIG. 16. FIG. 19 is a view explaining for displaying each of the library selection item, the data removal item, and a prosthesis generation item, adjacent to the artificial abutment model of FIG. 16. FIG. 20 is a view explaining for displaying the first tool menu for the scan body of FIG. 17. FIG. 21 is a view explaining for displaying the first tool meu for the artificial abutment model. FIG. 22 is a view explaining for displaying the second wheel menu for data removal operation and library selection, adjacent to the prosthesis of FIG. 15. FIG. 23 is a view explaining for the second tool menu providing items for generation and modification of the prosthesis of FIG. 15.

The method for providing the user interface described with reference to FIGS. 15, 16, 17, 18 19, 20, 21, 22, and 23 may be substantially a same as or similar to the method for providing the user interface described with reference to FIGS. 1, 2, 3, 4, 5, 6, 7, 8, 9, 10, 11, 12, 13, and 14, except for displaying the first wheel menu 200 adjacent to a scan body 112 or an artificial abutment model 114 S400A, displaying the first tool menu 300 providing items for generating the prosthesis 162 for the scan body 112 or the artificial abutment model 114 S500A, displaying the second wheel menu 220 for data removal operation and library selection, adjacent to the prosthesis 162 S700A, and displaying the second tool menu 400 providing items for generation and modification of the prosthesis 162 S800A.

Referring to FIGS. 15, 16, 17, 18, 19, 20, 21, 22, and 23, a method for providing a user interface for manufacturing a prosthesis according to another embodiment of the present inventive concept may include identifying teeth from the three-dimensional oral data of the patient and determining the type of a prosthesis S100, generating a margin line of a target tooth for dental work S200, displaying the target tooth for dental work on a screen in the selectable state by a user S300, displaying the first wheel menu 200 adjacent to a scan body 112 or an artificial abutment model 114 S400A, displaying the first tool menu 300 providing items for generating the prosthesis 162 for the scan body 112 or the artificial abutment model 114 S500A, generating the prosthesis to be combined with the target tooth for dental work, based on data set in the first wheel menu and the first tool menu S600, displaying the second wheel menu 220 for data removal operation and library selection, adjacent to the prosthesis 162 S700A, and displaying the second tool menu 400 providing items for generation and modification of the prosthesis 162 S800A.

The displaying the first wheel menu 200 adjacent to the scan body 112 or the artificial abutment model 114 S400A may include displaying a library selection item and a data removal item adjacent to the scan body S420A, generating the artificial abutment model based on values in the library selection item, and displaying each of the library selection item, the data removal item, and a prosthesis generation item, adjacent to the artificial abutment model.

The displaying the first tool menu 300 providing items for generating the prosthesis 162 for the scan body 112 or the artificial abutment model 114 S500A may include displaying a first tool menu for the scan body S520A and displaying the first tool menu for the artificial abutment model S540A.

In the displaying the target tooth for dental work on the screen in the selectable state by the user S300, the controller (e.g., the controller 14 of FIG. 2) may extract a scan body 112, which is implanted in the oral cavity for implant treatment, from the three-dimensional oral data of the patient, and the output part (e.g., the output part 18 of FIG. 2) may display the scan body 112 on the screen.

In the displaying the library selection item and the data removal item adjacent to the scan body S420A, the user may directly select the image of the scan body 112 or select the tooth number label 120 indicating the tooth identification number of the scan body 112. When the user selects the image or the tooth number label 120 of the scan body 112, the library selection item and the data removal item may be displayed around the tooth number label 120 of the selected scan body 112. In other words, when the user selects the scan body 112, the first wheel menu 200 displayed adjacent to the scan body 112 may provide the library selection item and the data removal item.

The user may perform an operation to directly set the artificial abutment model 114 to replace the scan body 112 using the library selection item. For example, when the user selects the library selection item, the user may select the company, product type, material, and the like of the artificial abutment model 114. The user may perform an operation to delete data set to the scan body 112 during the extraction of the scan body 112 from the three-dimensional oral data by the controller, using the data removal item. However, operations performed using the library selection item and the data removal item according to the embodiments of the inventive concept may not be limited thereto.

When the user inputs all necessary values to generate the artificial abutment model 114 using the library selection item, the generating the artificial abutment model 114 based on the values input in the library selection item S440A may be performed. In the generating the artificial abutment model 114 based on the values input in the library selection item S440A, a process of aligning the artificial abutment model 114 to be accurately arranged in the three-dimensional oral data may be performed. After the artificial abutment model 114 is generated on the screen, the displaying each of the library selection item, the data removal item, and the prosthesis generation item, adjacent to the artificial abutment model 114 S460A may be performed.

In the displaying each of the library selection item, the data removal item, and the prosthesis generation item, adjacent to the artificial abutment model 114 S460A, the user may directly select the image of the artificial abutment model 114 or select the tooth number label 120 indicating the tooth identification number of the artificial abutment model 114. When the user selects the image or the tooth number label 120 of the generated abutment model 114, the library selection item, the data removal item, and the prosthesis generation item may be displayed around the tooth number label 120 of the selected abutment model 114. In other words, when the user selects the artificial abutment model 114, the first wheel menu 200 displayed adjacent to the artificial abutment model 114 may provide the library selection item, the data removal item, and the prosthesis generation item.

In the displaying each of the library selection item, the data removal item, and the prosthesis generation item, adjacent to the artificial abutment model 114 S460A, operations performed by each of the library selection item and the data removal item may be substantially a same as the operations performed by the library selection item and the data removal item in the displaying the library selection item and the data removal item adjacent to the scan body 112 S420A. The user may perform an operation to generate an implant crown to be combined with the artificial abutment model 114 using the prosthesis generation item.

Referring to FIGS. 20 and 21, the first tool menu 300 described with reference to FIGS. 20 and 21 may be substantially a same as or similar to the first tool menu 300 described with reference to FIGS. 9 and 10, except for the items activated, the classification type of the target tooth for dental work displayed in the first item 3100, and the identification number of the target tooth for dental work displayed in the first item 3100. Hereinafter, overlapping contents with those described with reference to FIGS. 9 and 10 will be omitted or simplified.

In the displaying the first tool menu 300 for the scan body 112 S520A, the first item 3100, the second item 3200, the third item 3300, and the fourth item 3400 may be displayed in the first tool menu 300. When the user selects the scan body 112, the classification type of the target tooth for dental work may be displayed as the implant in the first item 3100. The second item 3200 may be an item for the margin line (e.g., the margin line 130 of FIG. 3), the third item 3300 may be an item for the insertion direction of the prosthesis 162 to be combined with the abutment model 114, and the fourth item 3400 may be an item for the inner surface of the prosthesis 162.

In an embodiment, when the user selects the scan body 112, the first item 3100 and the fourth item 3400 may be activated to be in the selectable state in the first tool menu 300. In an embodiment, when the user selects the scan body 112, the second item 3200 and the third item 3300 may be deactivated to be in the non-selectable state in the first tool menu 300. In other words, in the displaying the first tool menu 300 for the scan body 112 S520A, the user may use the first tool menu 300 to modify data related to the tooth identification number and classification type according to the first item 3100, or modify data related to the inner surface of the prosthesis 162 (e.g., the implant crown) according to the fourth item 3400.

In an embodiment, the displaying the library selection item and the data removal item adjacent to the scan body 112 S420A and the displaying the first tool menu 300 for the scan body 112 S502A may be performed simultaneously. In other words, when the user selects the scan body 112, both of the first wheel menu 200 and the first tool menu 300 for the scan body 112 may be displayed on the screen.

In the displaying the first tool menu 300 for the abutment model 114 S540A, the first item 3100, the second item 3200, the third item 3300, and the fourth item 3400 may be displayed in the first tool menu 300. When the user selects the abutment model 114, the classification type of the target tooth for dental work may be displayed as the implant in the first item 3100. In an embodiment, when the user selects the abutment model 114, the first through fourth items 3100, 3200, 3300, and 3400 may all be activated to be in the selectable state in the first tool menu 300. In other words, in the displaying the first tool menu 300 for the abutment model 114 S540A, the user may use the first tool menu 300 to modify data related to the tooth identification number and classification type according to the first item 3100, modify data related to the margin line according to the second item 3200, modify data related to the insertion direction of the prosthesis 162 according to the third item 3300, or modify data related to the inner surface of the prosthesis 162 according to the fourth item 3400.

In an embodiment, the displaying each of the library selection item, the data removal item, and the prosthesis generation item, adjacent to the abutment model 114 S460A, and the displaying the first tool menu 300 for the abutment model 114 540A may be performed simultaneously. In other words, when the user selects the abutment model 114, both of the first wheel menu 200 and the first tool menu 300 for the abutment model 114 may be displayed on the screen.

In the displaying the second wheel menu 220 for data removal and library selection adjacent to the prosthesis 162 S700A, when the user selects the prosthesis 162, the library selection item and the data removal item may be displayed around the tooth number label 120. In other words, when the selected prosthesis 162 by the user is an implant crown, the second wheel menu 220 displayed adjacent to the prosthesis 162 may provide the library selection item and the data removal item.

The second tool menu 400 described with reference to FIG. 23 may be substantially a same as or similar to the second tool menu 400 described with reference to FIGS. 13 and 14, except for the items activated, the classification type of the prosthesis 162 displayed in the first item 4100, and the identification number of the target tooth for dental work displayed in the first item 4100. Hereinafter, overlapping contents with those described with reference to FIGS. 13 and 14 will be omitted or simplified.

In the displaying the second tool menu 400 for providing items for generation and modification of the prosthesis 162 800A, when the user selects the prosthesis 162, which is the implant crown, the classification type of the prosthesis 162 may be displayed as the implant in the first item 4100. In an embodiment, when the prosthesis 162 selected by the user is an implant crown, each of the first through seventh items 4100, 4200, 4300, 4400, 4500, 4600, and 4700 may be activated to be in the selectable state. In an embodiment, when the prosthesis 162 selected by the user is an implant crown, the eighth item 4800 may be deactivated to be in the non-selectable state.

As described above, in the method for providing the user interface for manufacturing the prosthesis according to the embodiments of the present inventive concept, the first wheel menu 200 for generating or removing data of the prosthesis 162 adjacent to the target tooth for dental work 110, the first tool menu 300 for providing items for generating the prosthesis 162 corresponding to the target tooth for dental work 110, the second wheel menu 220 for removing data adjacent to the prosthesis 162, and the second tool menu 400 for providing items for generating and modifying the prosthesis 162 may be displayed on the screen. Accordingly, the prosthesis manufacturing process may be performed quickly and accurately using the user interface providing method for manufacturing prosthesis. In addition, the prosthesis manufacturing process may be performed more efficiently using the first wheel menu 200, the second wheel menu 220, the first tool menu 300, and the second tool menu 400. In addition, since the first wheel menu 200, the second wheel menu 220, the first tool menu 300, and the second tool menu 400 provide a more intuitive and consistent user interface 100 for manufacturing the prosthesis, the efficiency of the prosthesis manufacturing process may be improved. In addition, the first wheel menu 200 and the second wheel menu 220 provide the library selection item for selecting the abutment model 114, so that an implant library based on user preference may be easily provided.

In the apparatus for providing the user interface for manufacturing the prosthesis 1 according to the embodiments of the present inventive concept, the controller 14 identifies teeth included in the three-dimensional oral data of a patient, automatically determines the type of prosthesis corresponding to each of the teeth, automatically generates the prosthesis 162 to be combined with the target tooth for dental work 110 based on data set in the first wheel menu 200 and the first tool menu 300, and the output unit 18 receives an output signal from the controller 14 and may display the first wheel menu 200, the second wheel menu 220, the first tool menu 300, and the second tool menu 400 on the screen. Accordingly, the user interface 100 and the apparatus for implementing the same may be provided to more efficiently perform the prosthesis manufacturing process.

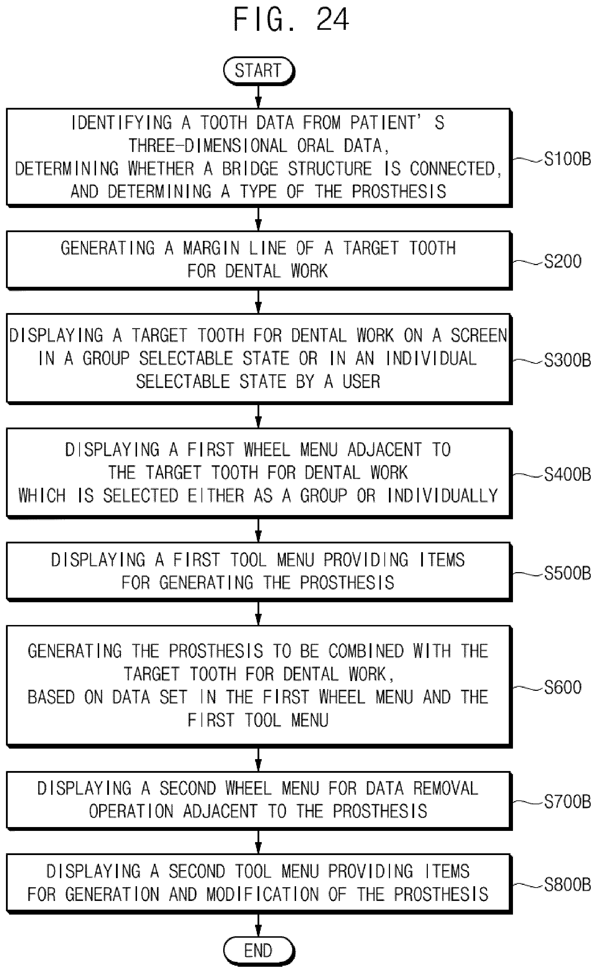

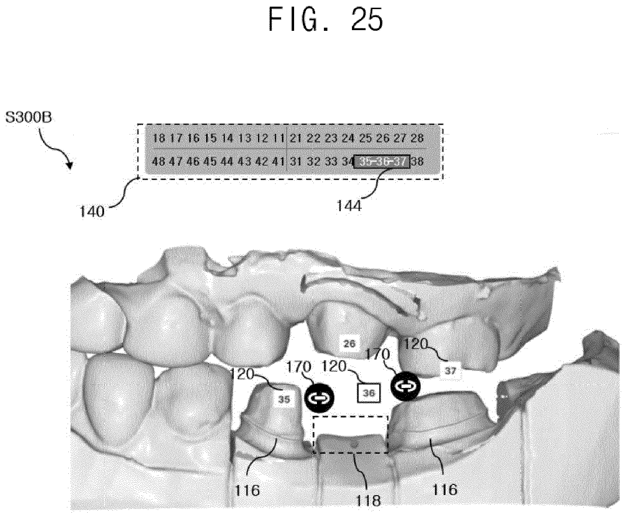

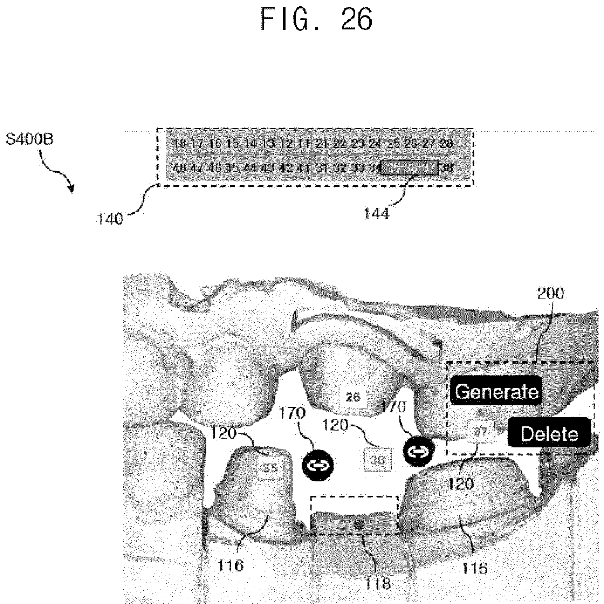

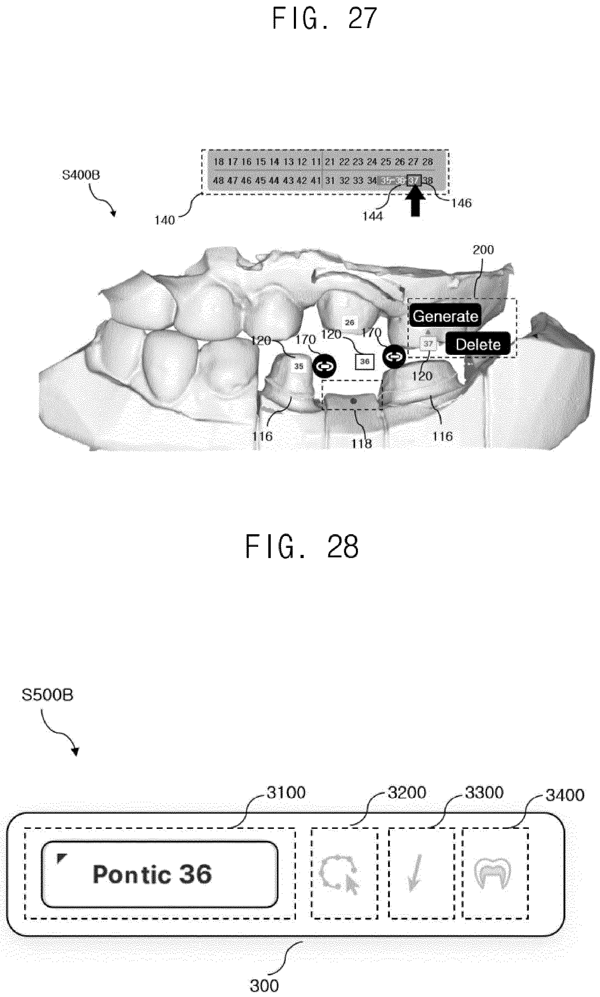

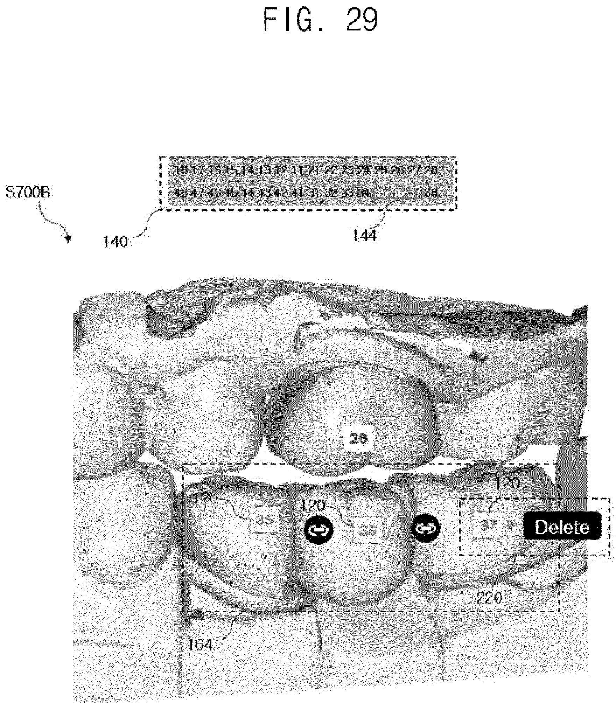

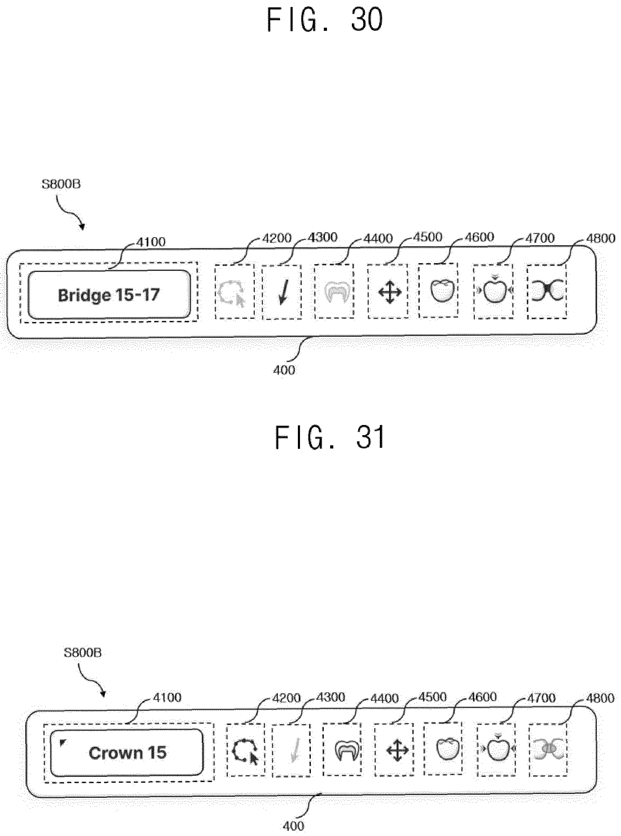

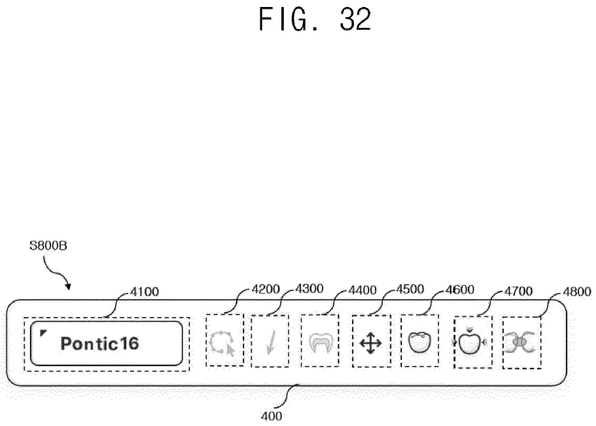

FIG. 24 is a flowchart illustrating a method for providing a user interface for manufacturing a prosthesis according to still another embodiment of the present inventive concept. FIG. 25 is a view explaining for displaying a target tooth for dental work on a screen in a group selectable state or in an individual selectable state by a user of FIG. 24. FIGS. 26 and 27 are views explaining for displaying the first wheel menu adjacent to the target tooth for dental work which is selected either as a group or individually of FIG. 24. FIG. 28 is a view explaining for displaying the first tool menu providing items for generating the prosthesis of FIG. 24. FIG. 29 is a view explaining for displaying the second wheel menu for data removal operation adjacent to the prosthesis of FIG. 24. FIG. 30 is a view explaining for an example of the second tool menu providing items for generation and modification of the prosthesis of FIG. 24. FIG. 31 is a view explaining for another example of the second tool menu providing items for generation and modification of the prosthesis of FIG. 24. FIG. 32 is a view explaining for still another example of the second tool menu providing items for generation and modification of the prosthesis of FIG. 24.

The user interface providing method described with reference to FIGS. 24, 25, 26, 27, 28, 29, 30, 31, and 32 may be substantially a same as or similar to the user interface providing method described with reference to FIGS. 1, 2, 3, 4, 5, 6, 7, 8, 9, 10, 11, 12, 13, and 14, except for identifying a tooth data from three-dimensional oral data of the patient, determining whether a bridge structure is connected, and determining a type of prosthesis S100B, displaying the target tooth for dental work on the screen in a group selectable state or in individual selectable state by the user S300B, displaying a first wheel menu 200 adjacent to the target tooth for dental work which is selected either as a group or individually S400B, displaying a first tool menu 300 for providing items for generating the prosthesis 164 S500B, displaying a second wheel menu 220 for data removal operation adjacent to the prosthesis 164 S700B, and displaying a second tool menu 400 for providing items for generation and modification of the prosthesis 164 S800B. Hereinafter, overlapping contents with the user interface providing method described with reference to FIGS. 1, 2, 3, 4, 5, 6, 7, 8, 9, 10, 11, 12, 13, and 14 will be omitted or simplified.

Referring to FIGS. 24, 25, 26, 27 28, 29, 30, 31, and 32, the method for providing the user interface for manufacturing the prosthesis according to still another embodiment of the present inventive concept may include identifying the tooth data from the three-dimensional oral data of the patient, determining whether the bridge structure is connected, and determining the type of prosthesis S100B, generating a margin line for the target tooth for dental work S200, displaying the target tooth for dental work on the screen in a group selectable state or in an individual selectable state by the user S300B, displaying the first wheel menu 200 adjacent to the target tooth for dental work which is selected either as a group or individually S400B, displaying the first tool menu 300 for providing items for generating the prosthesis 164 S500B, generating the prosthesis 164 to be combined with the target tooth for dental work, based on data set in the first wheel menu 200 and the first tool menu 300 S600, displaying the second wheel menu 220 for data removal operation adjacent to the prosthesis 164 S700B, and displaying the second tool menu 400 for providing items for generation and modification of the prosthesis 164 S800B.

In an embodiment, in the identifying the tooth data from the three-dimensional oral data of the patient, determining whether the bridge structure is connected, and determining the type of prosthesis S100B, the controller (e.g., the controller 14 of FIG. 2) may determine whether the bridge structure is connected between adjacent teeth among the teeth. For example, if the teeth that require dental treatment are arranged adjacent to each other, the controller may automatically determine whether to generate the bridge structure combining the teeth.

In the displaying the target tooth for dental work on the screen in the group selectable state or in the individual selectable state by the user S300B, tooth number label 120 of each of the target teeth for dental work adjacent to each other may be displayed on the screen.

In the displaying the target tooth for dental work on the screen in the group selectable state or in the individual selectable state by the user S300B, when the target teeth for dental work are adjacent teeth connected by the bridge structure, a group area 144 that allows group selection may be displayed within a tooth component 140. Specifically, when the controller determines that the bridge structure needs to be combined to two prepared teeth 116 and a missing tooth 118 located adjacent to the prepared teeth 116, a group area 144 that allows selection of two prepared teeth 116 and the missing tooth 118 at once may be activated for selection in the dental component 140. The user may perform group selection of the teeth corresponding to the bridge structure by selecting the group area 144.

The user may select an individual area 146 for the tooth identification number of one of the group areas 144, or select one of the teeth corresponding to the bridge structure. Accordingly, the user may perform individual selection for one of the teeth corresponding to the bridge structure.

In the displaying the target tooth for dental work on the screen in the group selectable state or in the individual selectable state by the user S300B, a link button 170 may be activated between adjacent target teeth. The user may perform a bridge connection operation or a bridge disconnection operation between the target teeth by selecting the link button 170.

In the displaying the first wheel menu 200 adjacent to the target tooth for dental work which is selected either as a group or individually S400B, when the user performs group selection of the target teeth corresponding to the bridge structure, the data removal item and the prosthesis generation item may be displayed around the tooth number label 120. In other words, the first wheel menu 200 may display each of the data removal item and the prosthesis generation item adjacent to the target teeth for dental work which are group-selected.

In the displaying the first wheel menu 200 adjacent to the target tooth for dental work which is selected either as a group or individually S400B, when the user individually selects one tooth among the target teeth for dental work corresponding to the bridge structure, the data removal item and the prosthesis generation item may be displayed around the tooth number label 120 of the individually selected tooth. In other words, the first wheel menu 200 may display each of the data removal item and the prosthesis generation item adjacent to the individually selected tooth.

The first tool menu 300 described with reference to FIG. 28 may be substantially a same as or similar to the first tool menu 300 described with reference to FIGS. 9 and 10, except for the items activated, the classification type of the target tooth displayed in the first item 3100, and the identification number of the target tooth displayed in the first item 3100. Hereinafter, overlapping contents with those described with reference to FIGS. 9 and 10 will be omitted or simplified.

In the displaying the first tool menu 300 for providing items for generating the prosthesis 164 S500B of displaying the first tool menu 300 for providing items for generating the prosthesis 164, the first item 3100, the second item 3200, the third item 3300, and the fourth item 3400 may be displayed in the first tool menu 300. In an embodiment, when the user individually selects the missing tooth 118 among the target teeth corresponding to the bridge structure, the classification type of the missing tooth 118 may be displayed as a pontic in the first item 3100. In an embodiment, when the user individually selects a prepared tooth among the target teeth corresponding to the bridge structure, the classification type of the prepared tooth may be displayed as crown in the first item 3100. In an embodiment, when the user individually selects the artificial abutment model(e.g., the artificial abutment model 114 of FIG. 19) for implant among the target teeth corresponding to the bridge structure, the classification type of the missing tooth 118 may be displayed as the implant in the first item 3100. The second item 3200 may be an item for the margin line (e.g., the margin line 130 of FIG. 3), the third item 3300 may be an item for the insertion direction of the prosthesis to be combined with the target teeth, and the fourth item 3400 may be an item for the inner surface of the prosthesis 164.

In an embodiment, when the individually selected target tooth is the missing tooth 118, the first item 3100 may be activated to be in the selectable state, and each of the second, third, and fourth items 3200, 3300, and 3400 may be deactivated to be in the non-selectable state. In an embodiment, when the individually selected target tooth is the prepared tooth or the abutment model (e.g., the abutment model 114 of FIG. 19), each of the first, second, third, and fourth items 3100, 3200, 3300, and 3400 may be activated to be in the selectable state.

In an embodiment, the displaying the first wheel menu 200 adjacent to the target tooth for dental work which is selected either as a group or individually S400B, and the displaying the first tool menu 300 for providing items for generating the prosthesis 164 S500B 164 may be performed simultaneously. In other words, when the user individually selects one of the target teeth corresponding to the bridge structure, both of the first wheel menu 200 and the first tool menu 300 for the selected tooth may be displayed. However, a displaying processes of each of the first wheel menu 200 and the first tool menu 300 according to embodiments of the present inventive concept may not be limited thereto and may also be performed simultaneously when the target teeth corresponding to the bridge structure are group-selected.

In the displaying the second wheel menu 220 for data removal operation adjacent to the prosthesis 164 S700B, the bridge structure may be displayed on the screen in a state combined with the target teeth. In an embodiment, the bridge structure may be individually selected or group-selected by the user. When the user group-selects the bridge structure, a data removal item may be displayed adjacent to the bridge structure. In other words, the second wheel menu 220 displayed adjacent to the group-selected prosthesis 164 may provide the data removal item.

The second tool menu 400 described with reference to FIGS. 30, 31, and 32 may be substantially a same as or similar to the second tool menu 400 described with reference to FIGS. 13 and 14, except for the items activated, the classification type of the prosthesis 164 displayed in the first item 4100, and the tooth identification number of the target tooth displayed in the first item 4100. Hereinafter, overlapping contents with those described with reference to FIGS. 13 and 14 will be omitted or simplified.

In the displaying the second tool menu 400 for providing items for generation and modification of the prosthesis 164 S800B, when the user group-selects the bridge structure, the classification type of the prosthesis 164 may be displayed as bridge in the first item 4100, and the tooth identification number of the prosthesis 164 may be displayed as the tooth identification numbers of the teeth combined with the bridge. In an embodiment, when the prosthesis 164 selected by the user is the group-selected bridge structure, the first item 4100, the third item 4300, the fifth item 4500, the sixth item 4600, the seventh item 4700, and the eighth item 4800 may each be activated in the selectable state. In an embodiment, when the prosthesis 164 selected by the user is the bridge structure, the second item 4200 and the fourth item 4400 may each be deactivated in the non-selectable state.

In the displaying the second tool menu 400 for providing items for generation and modification of the prosthesis 164 S800B, when the user individually selects a portion combined with the prepared tooth of the bridge structure, the classification type of the prosthesis 164 may be displayed as the crown in the first item 4100. In an embodiment, when the user individually selects a portion combined with the prepared tooth of the bridge structure, the first item 4100, the second item 4200, the fourth item 4400, the fifth item 4500, the sixth item 4600, and the seventh item 4700 may each be activated in the selectable state. In an embodiment, when the user individually selects a portion combined with the prepared tooth of the bridge structure, the third item 4300 and the eighth item 4800 may each be activated in the selectable state.

In the displaying the second tool menu 400 for providing items for generation and modification of the prosthesis 164 S800B, when the user individually selects a portion combined with the missing tooth of the bridge structure, the classification type of the prosthesis 164 may be displayed as the pontic in the first item 4100. In an embodiment, when the user individually selects a portion combined with the missing tooth of the bridge structure, the first item 4100, the fifth item 4500, the sixth item 4600, and the seventh item 4700 may each be activated in the selectable state. In an embodiment, when the user individually selects a portion combined with the prepared tooth of the bridge structure, the second item 4200, the third item 4300, the fourth item 4400, and the eighth item 4800 may be activated in the selectable state.

In the displaying the second tool menu 400 for providing items for generation and modification of the prosthesis 164 S800B, when the user individually selects a portion combined with the abutment model for implant of the bridge structure, the classification type of the prosthesis 164 may be displayed as the implant in the first item 4100. In an embodiment, when the user individually selects a portion combined with the abutment model for implant of the bridge structure, the first item 4100, the second item 4200, the fourth item 4400, the fifth item 4500, the sixth item 4600, and the seventh item 4700 may each be activated in the selectable state. In an embodiment, when the user individually selects a portion combined with the abutment model for implant of the bridge structure, the third item 4300 and the eighth item 4800 may each be activated in the selectable state.

As described above, in the method for providing the user interface for manufacturing the prosthesis to embodiments of the present inventive concept, the first wheel menu 200 for generating or removing data of the prosthesis 164 adjacent to the target tooth 110, the first tool menu 300 for providing items for generating the prosthesis 164 corresponding to the target tooth 110, the second wheel menu 220 for removing data adjacent to the prosthesis 164, and the second tool menu 400 for providing items for generating and modifying the prosthesis 164 may be displayed on the screen. Accordingly, the prosthesis manufacturing process may be performed quickly and accurately by using the user interface providing method for manufacturing the prosthesis. In addition, the prosthesis manufacturing process may be further accelerated using the first wheel menu 200, the second wheel menu 220, the first tool menu 300, and the second tool menu 400. In addition, since the first wheel menu 200, the second wheel menu 220, the first tool menu 300, and the second tool menu 400 provide a more intuitive and consistent user interface 100 for manufacturing the prosthesis, the efficiency of the prosthesis manufacturing process may be improved. In addition, since individual selection or group selection of the bridge structure is possible within the user interface 100, the time required for the bridge structure manufacturing process may be reduced, and the efficiency of the manufacturing process may be improved.

In the apparatus for providing the user interface for manufacturing the prosthesis 1 according to embodiments of the present inventive concept, the controller 14 may identify the teeth included in the three-dimensional oral data of the patient, automatically determine the type of prosthesis corresponding to each tooth, automatically generate the prosthesis 162 to be combined with the target tooth 110 based on the data set in the first wheel menu 200 and the first tool menu 300, and the output unit 18 may receive an output signal from the controller 14 and display the first wheel menu 200, the second wheel menu 220, the first tool menu 300, and the second tool menu 400 on the screen. Accordingly, the user interface 100 and the apparatus for implementing the same may be provided, which enable the prosthesis manufacturing process to proceed more efficiently.

According to an embodiment of the present inventive concept, a computer readable medium having recorded thereon a program for executing the method for providing a user interface for manufacturing the prosthesis according to the above embodiments on a computer may be provided. The above-described method may be written as a program executed on the computer, and may be implemented on a general-purpose digital computer that executes the program using the computer readable medium. In addition, a structure of data used in the above-described method may be recorded on the computer readable medium through various means. The computer readable medium may include program commands, data files, data structures, and the like, either singly or in combination. The program commands recorded on the medium may be those specially designed and configured for the present inventive concept, or may be known and usable by those skilled in the art in the field of computer software. Examples of computer readable recording media include magnetic media such as hard disks, floppy disks, and magnetic tapes; optical recording media such as CD-ROMs and DVDs; magneto-optical media such as floptical disks; and hardware devices specially configured to store and execute program commands such as ROMs, RAMs, and flash memories. Examples of program instructions include not only machine language codes, such as those generated by a compiler, but also high-level language codes that can be executed by a computer using an interpreter or the like. The hardware devices described above may be configured to operate as one or more software modules to perform the operations of the present inventive concept.

The present inventive concept relates to the method and apparatus for providing the user interface for manufacturing the prosthesis, and the non-transitory computer readable medium having the program recorded for performing the method, and the method, the apparatus, and the medium may reduce the effort and time required for prosthesis fabrication and may improve the accuracy and productivity of the prosthesis.

While the above has been described with reference to exemplary embodiments of the present inventive concept, it will be understood by those skilled in the art that the present inventive concept may be variously modified and changed without departing from the spirit and scope of the present inventive concept as defined in the following claims.

## Claims

1. A method for providing a user interface for manufacturing a prosthesis, the method comprising:
identifying teeth from a three-dimensional oral data of a patient;
displaying a target tooth for a prosthesis manufacturing among the teeth on a screen in a selectable state by a user;
displaying a first wheel menu for generation of the prosthesis for the target tooth or data removal operation; and
displaying a first tool menu providing items for the generation of the prosthesis corresponding to the target tooth on the screen.

2. The method of claim 1, wherein the displaying the target tooth on the screen in the selectable state by the user includes:
displaying a tooth identification number of the target tooth in the selectable state in a tooth number component including the tooth identification number of each of the teeth;
displaying a three-dimensional image of the three-dimensional oral data on the screen in a three-dimensional rotatable state; and
displaying a tooth number label for the tooth identification number of the target tooth included in the three-dimensional image adjacent to the target tooth.

3. The method of claim 1, wherein in the displaying the first tool menu on the screen, when the target tooth is selected by the user, a first item for the tooth identification number of the target tooth and a classification type of the target tooth, a second item for a margin line of the target tooth, a third item for an insertion direction of the prosthesis, and a fourth item for an inner surface of the prosthesis are displayed in the first tool menu.

4. The method of claim 3, wherein the displaying the first wheel menu on the screen includes:
displaying a library selection item and a data removal item adjacent to a scan body;
generating an artificial abutment model based on values input in the library selection item; and
displaying the library selection item, the data removal item, and a prosthesis generation item adjacent to the artificial abutment model.

5. The method of claim 4, wherein in the displaying the first tool menu on the screen, when the scan body is selected by the user, the classification type is displayed as an implant in the first item, and the first item and the fourth item are active in the selectable state, and the second item and the third item are inactive in a non-selectable state.

6. The method of claim 4, wherein in the displaying the first tool menu on the screen, when the artificial abutment model is selected by the user, the classification type corresponding to the target tooth is displayed as an implant in the first item, and the first item, the second item, the third item, and the fourth item are active in the selectable state.

7. The method of claim 3, further comprising:
determining a type of the prosthesis corresponding to each of the teeth,
wherein when the prosthesis corresponding to the target tooth is bridge structure, in the determining the type of the prosthesis corresponding to each of the teeth, whether the bridge structure between adjacent teeth among the teeth is connected is determined, and in the displaying the target tooth on the screen in the selectable state by the user, the target tooth is displayed in a group selectable state or in an individual selectable state.

8. The method of claim 7, wherein in the displaying the first tool menu on the screen, when a missing tooth combined with the bridge structure is selected by the user, the classification type is displayed as a pontic in the first item, the first item is active in the selectable state, and the second item, the third item, and the fourth item are inactive in a non-selectable state.

9. The method of claim 1, further comprising:
generating the prosthesis combined with the target tooth based on a data set in the first wheel menu and the first tool menu;
displaying a second wheel menu for data removal operation adjacent to the prosthesis; and
displaying a second tool menu providing items for generation and modification of the prosthesis.

10. The method of claim 9, wherein in the displaying the second tool menu, when the prosthesis is selected by the user, a first item for a tooth identification number of the target tooth combined with the prosthesis and a classification type of the prosthesis, a second item for a margin line of the target tooth, a third item for an insertion direction of the prosthesis, a fourth item for an inner surface of the prosthesis, a fifth item for three-dimensional manipulation of the prosthesis, a sixth item for modification of an outer shape of the prosthesis, a seventh item for adjustment between the prosthesis and an adjacent tooth or an opposite tooth of the prosthesis, and an eighth item for a bridge connection of the prosthesis are displayed in the second tool menu.

11. An apparatus for providing a user interface for manufacturing a prosthesis, the apparatus comprising:
an input part configured to receive a manipulation signal of a user;
a controller configured to identify teeth included in a three-dimensional oral data of a patient, and extract items for a generation and a modification of the prosthesis corresponding to a target tooth for dental work among the teeth, according to the manipulation signal received from the input part; and
an output part configured to receive an output signal from the controller, and display a three-dimensional image of the three-dimensional oral data, a wheel menu displayed adjacent to the target tooth, and a tool menu providing the items for the generation or the modification of the prosthesis.

12. The apparatus of claim 11, wherein the wheel menu includes:
a first wheel menu configured to be displayed adjacent to the target tooth; and
a second wheel menu configured to be displayed adjacent to the prosthesis combined with the target tooth.

13. The apparatus of claim 11, wherein the tool menu includes:
a first tool menu configured to provide a first item, a second item, a third item, and a fourth item for the generation of the prosthesis; and
a second tool menu configured to provide the first item, the second item, the third item, and the fourth item for the generation of the prosthesis, and configured to provide a fifth item, a sixth item, a seventh item, and an eighth item for the modification of the prosthesis.

14. The apparatus of claim 13, wherein the first item provides a modification of a tooth identification number of the target tooth and a classification type of the target tooth or the prosthesis,
wherein the second item provides a modification of a margin line of the target tooth,
wherein the third item provides a modification of an insertion direction of the prosthesis,
wherein the fourth item provides a modification of an inner surface of the prosthesis,
wherein the fifth item provides a three-dimensional manipulation of the prosthesis,
wherein the sixth item provides a modification of an outer shape of the prosthesis,
wherein the seventh item provides a modification for adjustment between the prosthesis and an adjacent tooth of an opposite tooth of the prosthesis, and
wherein the eighth item provides a modification for a bridge connection of the prosthesis.

15. A non-transitory computer-readable storage medium having stored thereon program instructions, the program instructions executable by at least one hardware processor to:
identify teeth from a three-dimensional oral data of a patient;
display a target tooth for a prosthesis manufacturing among the teeth on a screen in a selectable state by a user;
display a first wheel menu for generation of the prosthesis for the target tooth or data removal operation; and
display a first tool menu providing items for generation of prosthesis corresponding to the target tooth on the screen.
